# EUROPEAN PATENT SPECIFICATION

(11) **EP 2 847 213 B1**
(45) Date of publication and mention of the grant of the patent: **03.01.2018**
(21) Application number: 13723718.6
(22) Date of filing: 07.05.2013
(51) Int. Cl.: C07K 14/435

(54) **ANTI-INFLAMMATORY PEPTIDES AND COMPOSITION COMPRISING THE SAME**
ENTZÜNDUNGSHEMMENDE PEPTIDE UND ZUSAMMENSETZUNG DAMIT
PEPTIDES ANTI-INFLAMMATOIRES ET COMPOSITION COMPRENANT CEUX-CI

(30) Priority: 11.05.2012 KR 20120050529; 11.05.2012 KR 20120050533; 02.07.2012 KR 20120071989; 19.09.2012 KR 20120104144; 19.09.2012 KR 20120104207; 15.03.2013 WO PCT/EP2013/055329
(43) Date of publication of application: 18.03.2015
(73) Proprietor: KAEL-GemVax Co.,Ltd, Gyeonggi-do 463-440 (KR); Kim, Sang Jae, Gyeonggi-do 463-440 (KR)
(72) Inventor: KIM, Sang Jae, Seongnam-si Gyeonggi-do 463-440 (KR); KIM, Kyung Hee, Seongnam-si Gyeonggi-do 463-440 (KR); LEE, Kyu-Yong, Seoul 133-070 (KR); KOH, Seong-Ho, Seoul 133-070 (KR); PARK, Hyun-Hee, Seoul 133-070 (KR); HUH, Sung Jin, Seongnam-si Gyeonggi-do 463-440 (KR); LEE, Woo Jin, Seongnam-si Gyeonggi-do 463-440 (KR); KIM, Bum Joon, Seoul 110-744 (KR)
(74) Representative: Inspicos P/S
(86) International application number: PCT/EP2013/059460
(87) International publication number: WO 2013/167574

(56) References cited:
- WO-A1-00/02581
- WO-A1-2011/101173
- WO-A2-2010/003520
- US-A1- 2007 190 561
- MARTINEZ PAULA ET AL: "Telomeric and extra-telomeric roles for telomerase and the telomere-binding proteins", NATURE REVIEWS CANCER,, vol. 11, no. 3, 1 March 2011 (2011-03-01), pages 161-176, XP002696628,

## Description

### FIELD OF THE INVENTION

The present invention relates to the use of anti-inflammatory peptides and compositions comprising the same in treatment or prevention of inflammatory disease.

### BACKGROUND OF THE INVENTION

Inflammation is a type of biological defense as a means of protecting the body from damage of biological tissues that could be caused by external physical stimuli, chemical stimuli such as exposure to various allergens, or invasion of microorganisms including bacteria, fungi and viruses.

The Cyclooxygenase(COX) pathway or Lipoxygenase(LOX) Pathway can used for signaling inflammation, which produce prostaglandin, thromboxane, etc. Once the inflammatory signal is delivered, one of many changes that happen in the body is the expansion of the blood vessel for increased blood supply around the inflammation to concentrate blood cells such as neutrophils required for the inflammatory response. However, inflammatory diseases can result if an abnormal biological defense response occurs excessively. To prevent this, drugs that suppress excessive inflammatory responses by repressing enzymes used in inflammatory signaling pathway (for example, COX-1, COX-2, 5-LOX, 12-LOX etc.) are under development. According to response time, inflammation is categorized as acute inflammation (immediate response, non-specific response, several days to several weeks), chronic inflammation (delayed response, specific response, several weeks or more), subacute inflammation(a middle stage in between acute inflammation and chronic inflammation, characteristics of mixed product of mononuclear and polymorphounuclear).

Also, aside from peptide factors, factors such as prostaglandin, leukotriene, lipid factors including platelet activating factor(PAF), synthetic enzyme of inflammation factor, free radical such as NO(nitric oxide), many kinds of cell adhesion molecules, the immune system, and coagulation factors can cause inflammation.

Once a cell is damaged due to the known causative agents of inflammation such as external biological factors (microbes, viruses, parasites), physical factors (mechanical stimuli, heat, radiation, electricity), and chemical factors, histamine and kinin are released. The released histamine and kinin will result in angiectasis, increased capillary permeability and concentration of macrophages at the inflammation site, and it causes increased blood flow rate, edema, immunocyte and antibody migration, pain and heat generation.

Currently used treatments for inflammation are synthetic drugs such as ibuprofen, antihistamines, steroids, cortisone, immunosuppressive agents, and immune agonist; those which only temporarily alleviate inflammation. These drugs do not fundamentally cure inflammation, and they have side effects such as hypersensitivity reaction, and deterioration of immune system,

Therefore, for effective alleviation of inflammation, research is conducted to develop a substance that inhibits expression of the above mentioned inflammatory proteins. However, problems have arisen in anti-inflammation substances that had been developed previously. Diverse categories of anti-inflammatory drugs including Non-steroidal Anti-inflammatory Drugs (NSAIDs) and Steroidal Anti-inflammatory Drugs (SAIDs) have been developed; but not only do these drugs often bear side effects upon use, they also do not fundamentally cure the inflammation. Thus, there is a current need for anti-inflammatory drugs that are both physically and economically feasible. As one example, in acute or chronic inflammations such as chronic rheumatoid arthritis, not only do non-steroidal anti-inflammatory drugs suppress COX-2 enzyme activity, they are also known to suppress COX-1 activity, causing side effects such as gastrointestinal disorders.

The present invention was completed as present inventors have found that peptides derived from telomerase can have anti-inflammatory properties.

Therefore the objective of this invention is to provide an anti-inflammatory composition that uses this peptide as an active ingredient.

Another objective of present invention is to provide a pharmaceutical composition that uses this peptide as an active ingredient.

### SUMMARY OF THE INVENTION

In one embodiment of the present invention, the use of a peptide with anti-inflammatory activity, wherein the peptide comprises of amino acid sequence of SEQ ID NO: 1, or where the peptide has at least 80% homology with the amino acid sequence with SEQ ID NO: 1, or the peptide is a fragment of the above-mentioned peptides, in treatment or prevention of inflammatory disease is provided.

In another embodiment, the above-mentioned peptide consists of 30 or less amino acids.

In another embodiment of the composition, the above-mentioned composition is a pharmaceutical composition for treatment or prophylaxis of inflammatory disease.

In another embodiment of the composition, the above-mentioned composition is a food composition for treatment or prophylaxis of inflammation.

In another embodiment of the composition, the above-mentioned inflammatory disease is characterized by selecting from the group consisting of (1) general or localized inflammatory disease (for example, allergies; immune-complex disease; hayfever; hypersensitive shock; endotoxin shock; cachexia, hyperthermia; granulomatosis; or sarcoidosis); (2) gastro-intestinal related diseases (for example, appendicitis; gastric ulcer; duodenal ulcer; peritonitis; pancreatitis; ulcerative, acute, or ischemic colitis; cholangitis; cholecystitis, steatorrhea, hepatitis, Crone's disease; or Whipple's Disease); (3) dermal related diseases (for example, psoriasis; burns; sunburns; dermatitis; Urticarial warts or wheal); (4) vascular related diseases (for example, angiitis; vasculitis; endocarditis; arteritis; atherosclerosis; thrombophlebitis; pericarditis; congestive heart failure; myocarditis; myocardial ischemia; periarteritis nodosa; recurrent stenosis; Buerger's disease; or rheumatic fever); (5) respiratory diseases (for example, asthma; epiglottitis; bronchitis; emphysema; rhinitis; cystic fibrosis; interstitial pneumonitis; COPD(chronic obstructive pulmonary disease); adult respiratory distress syndrome; coniosis; alveolitis; bronchiolitis; pharyngitis; pleurisy; or sinusitis); (6) bone, joint, muscle and connective tissue related diseases (for example, eosinophilic granuloma; arthritis; arthralgia; osteomyelitis; dermatomyositis; fasciitis; Paget's disease; gout; periodontal disease; rheumatoid arthritis; myasthenia gravis; ankylosing spondylitis; or synovitis); (7) urogenital disorders (for example, epididymitis; vaginitis; prostatitis; or urethritis); meningitis; encephalitis; multiple sclerosis; cerebral infarction; cerebral embolism; Guillain-Barre syndrome; neuritis; neuralgia; spinal cord injury; paralysis; or uveitis); infectious disease (for example, Dengue fever).

In one embodiment of the present invention, a method for treating or preventing inflammatory diseases by administering the anti-inflammatory composition is provided.

In one embodiment of the present invention, a kit for prophylaxis or treatment of inflammatory disease comprising: a peptide with anti-inflammatory activity or a composition comprising the peptide, wherein the peptide comprises amino acid sequence of SEQ ID NO: 1, the peptide has above 80% amino acid sequence homology with SEQ ID NO: 1, or the peptide is a fragment of above-mentioned peptides; and instructions including at least one of administration dose, administration route, administration frequency, and indication of the peptide or composition, is provided.

### INDUSTRIAL APPLICABILITY

According to the present invention, a peptide that has a sequence of SEQ ID NO: 1 has outstanding efficacy in both suppressing inflammation and in prophylactic means. Therefore, the composition comprising the peptides of this invention can be used as anti-inflammatory pharmaceutical composition or as cosmetic composition, in turn, treating and preventing a variety of different types of inflammatory diseases.

### REFERENCE

KR2012-0130996A
KR2012-0133661A
KR2011-0060940A
US2011-0150873A1
Bonaldi T et al., EMBO J, (22)5551-60, 2003
Yankner BA et al, Science (New York, N.Y.) [1990, 250(4978):279-282]
Dahlgren KN et al, J. Biol. Chem. 277:32046-32053, 2002.

### BRIEF DESCRIPTION OF THE DRAWINGS

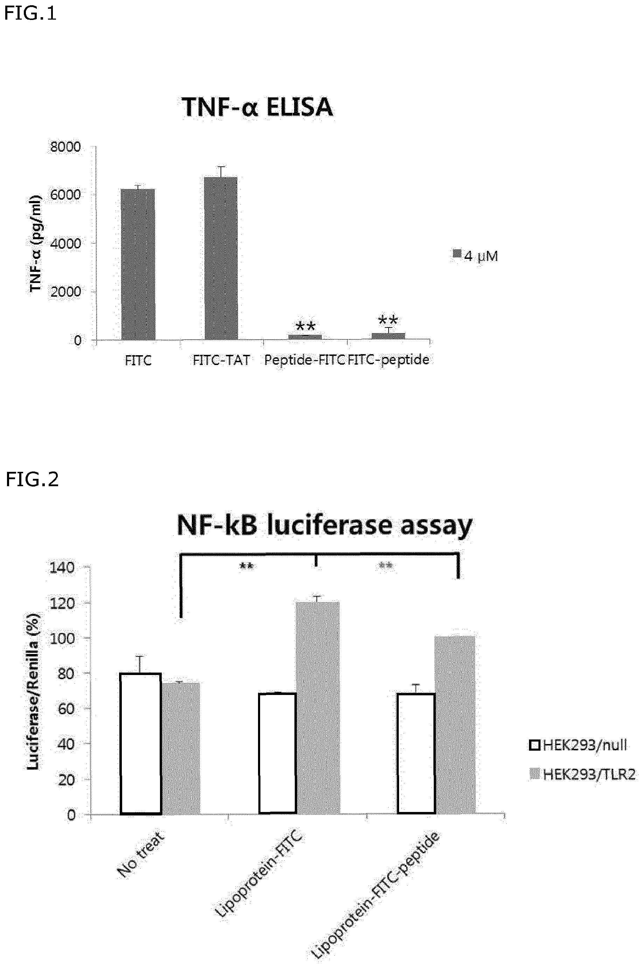

Figure 1 is a graph which shows the results of performing TNF-α ELISA with the culture of monocytes derived from PBMC. The monocytes were stimulated with LPS (10 ng/ml) for two hours, then reacted with each peptide, FITC, FITC-TAT, PEP 1-FITC and FITC-peptide for two hours. (** P < 0.01. Compared with the negative control (FITC and FITC-TAT).
Figure 2 is a graph which shows the results of performing luciferase analysis from transfecting HEK293/null and HEK293/TLR2 cell lines with NF-kB luciferase, then reacting with lipoprotein (10 ng/ml) and FITC and FITC-PEP-1(4 µM), and incubating for 18 hours. Results of luciferase were obtained by correction using renilla. (** P < 0.01. compared to the negative control (No treat) and compared with lipoprotein treated sample.)

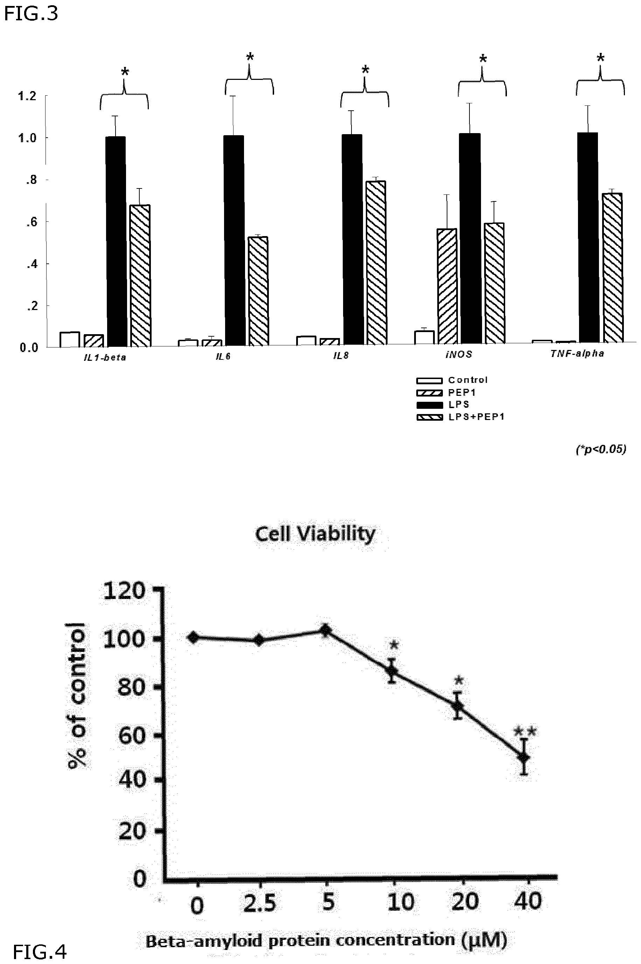

FIG. 3 represents the inhibition levels of cytokines in THP1 cell line which were treated with none, LPS, PEP 1 and LPS+PEP 1.
FIG. 4 represents viability of neural stem cell treated with 0, 2.5, 5.0, 10, 20 and 40 µM of amyloid-β protein.

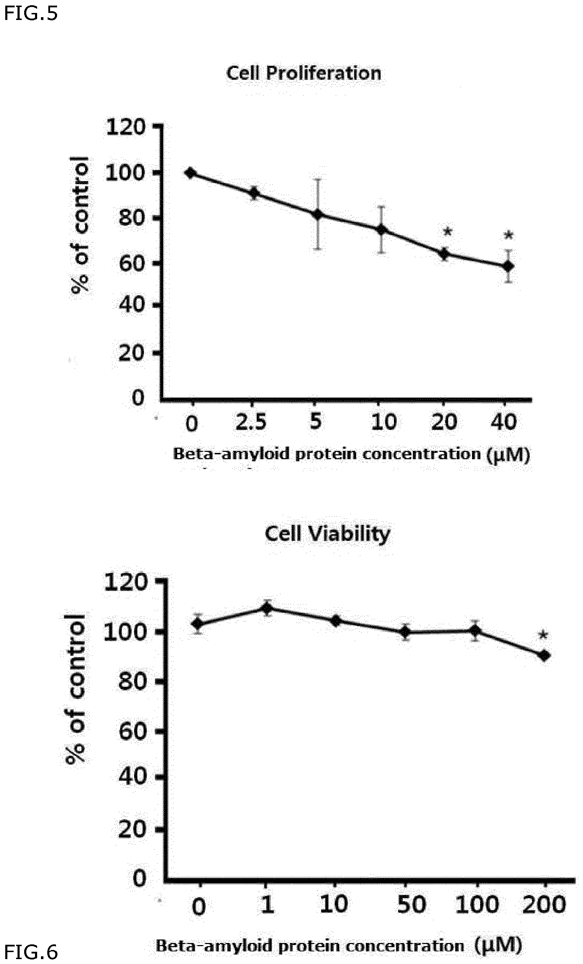

FIG. 5 represents proliferation of neural stem cell treated with 0, 2.5, 5.0, 10, 20 and 40 µM amyloid-β protein.
FIG. 6 represents viability of neural stem cell treated with 0, 1, 10, 50, 100 and 200µM of PEP 1.

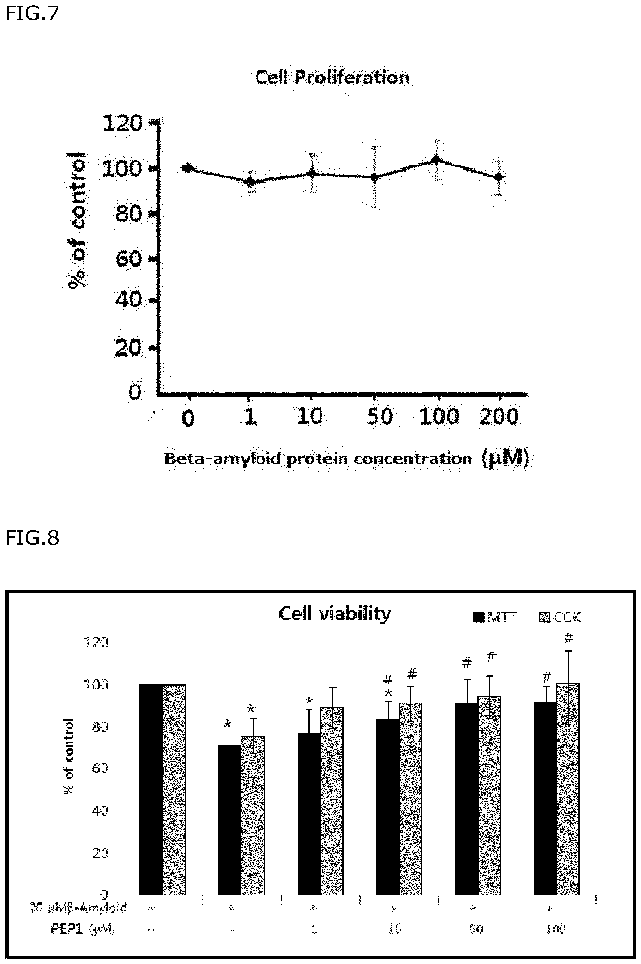

FIG. 7 represents proliferation of neural stem cell treated with 0, 1, 10, 50, 100 and 200µM of PEP 1.
FIG. 8 represents viability of neural stem cell treated with1, 10, 50 and 100µM PEP 1; neural stem cells were damaged by 20µM of amyloid beta protein, and then cell viability was measured after treatment with different concentrations of PEP-1. (Control groups were those untreated with amyloid beta protein and telomerase-based peptides).

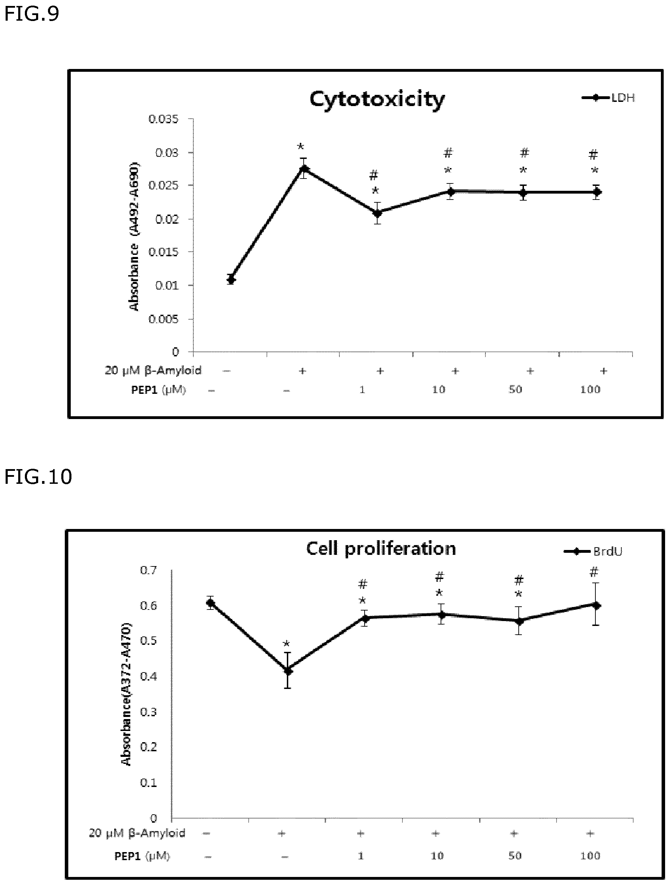

FIG. 9 represents toxicity of neural stem cell treated with1, 10, 50 and 100µM PEP 1; neural stem cells were damaged by 20µM of amyloid beta protein, and then cell toxicity was measured after treatment with different concentrations of PEP-1. (Control groups were those untreated with amyloid beta protein and telomerase-based peptides).
FIG. 10 represents proliferation of neural stem cells treated with1, 10, 50 and 100µM PEP 1; neural stem cells were damaged by 20µM of amyloid beta protein, and then cell proliferation was measured after treatment with different concentrations of PEP-1. (Control groups were those untreated with amyloid beta protein and telomerase-based peptides).

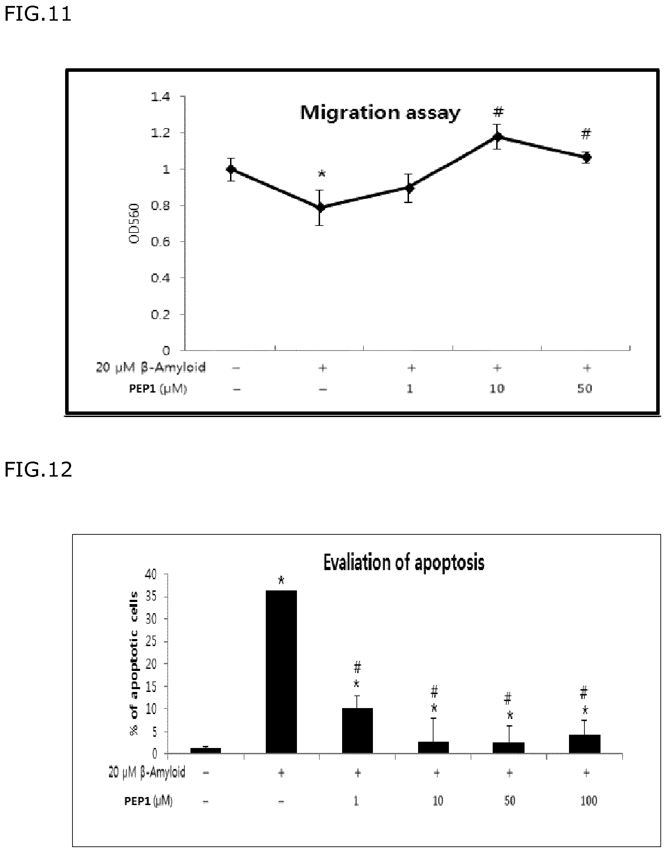

FIG. 11 represents migration of neural stem cells treated with1, 10, 50 and 100µM PEP 1; neural stem cells were damaged by 20µM of amyloid beta protein, and then cell migration was measured after treatment with different concentrations of PEP-1. (Control groups were those untreated with amyloid beta protein and PEP-1).
FIG. 12 represents apoptosis of neural stem cells treated with1, 10, 50 and 100µM PEP 1; neural stem cells were damaged by 20µM of amyloid beta protein, and then cell apoptosis was measured after treatment with different concentrations of PEP-1. (Control groups were those untreated with amyloid beta protein and telomerase-based peptides).

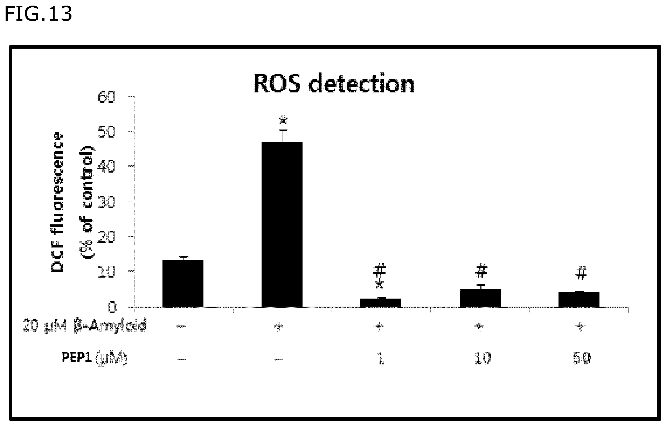

FIG. 13 represents ROS (Reactive Oxygen Species) inhibitory effect of PEP-1 in neural stem cells damaged by amyloid beta peptide; neural stem cells were damaged by 20µM of amyloid beta protein, and then inhibition of ROS was measured after treatment with different concentrations of PEP-1(1, 10, 50 and 100µM). (Control groups were those untreated with amyloid beta protein and PEP-1).

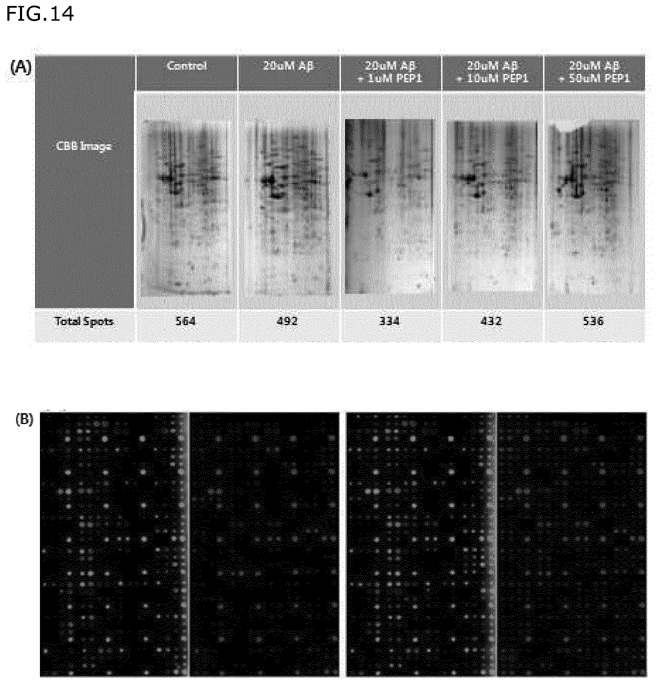

FIG. 14 represents the results of protein expression levels analyzed by (A) 2D-eletrophoresis and (B) Antibody Array; neural stem cells were damaged by 20µM of amyloid beta protein, and then protein expression level was measured after treatment with different concentrations of PEP-1(1, 10 and 50µM). (Control groups were those untreated with amyloid beta protein and PEP-1).

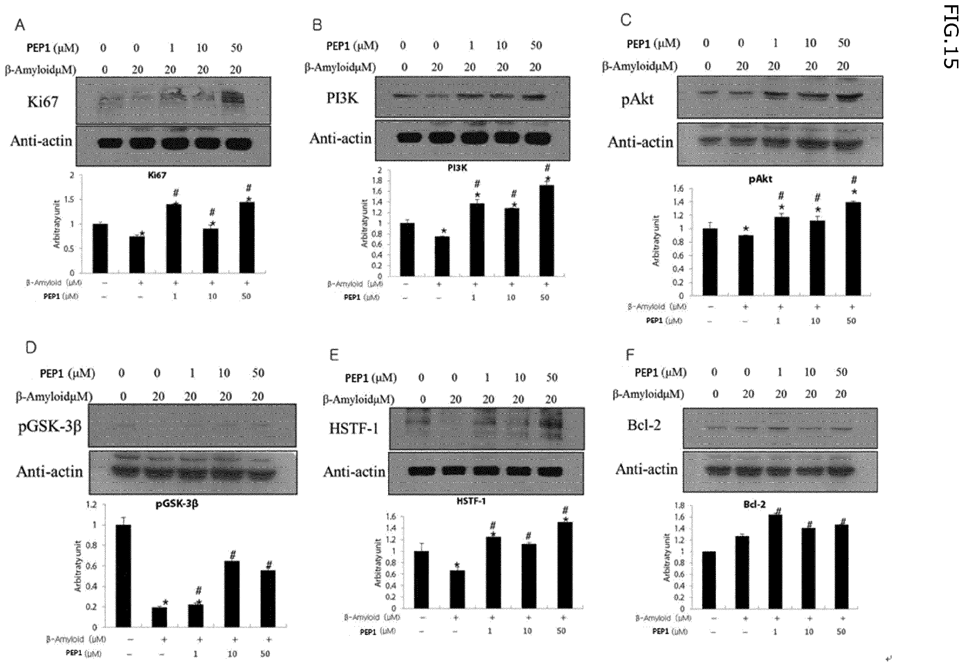

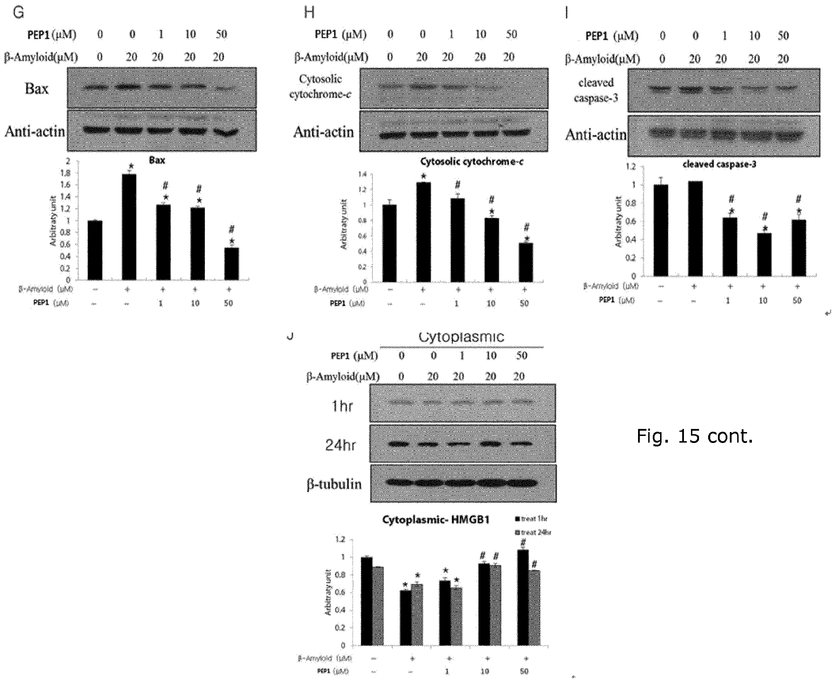

FIG. 15 represents the results of western blot showing the expression level of inflammation-related proteins: neural stem cells were damaged by 20µM of amyloid beta protein, and then cells were treated with different concentrations of PEP-1(1, 10 and 50µM).

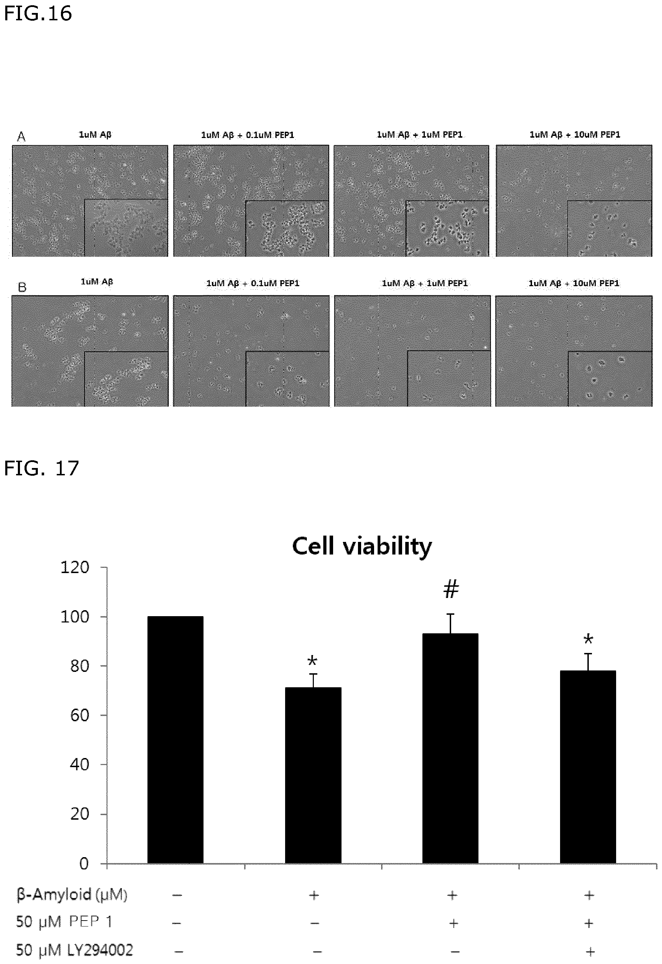

FIG. 16 represents the inhibitory effect of PEP 1 on amyloid beta protein aggregation; (A) shows the reduced oligomerization of amyloid beta proteins when co-treated with 1µM amyloid beta protein and PEP 1 (0.1, 1 and 10µM). (B) shows the case when PEP-1 was treated on the amyloid-β protein that was already induced for aggregation.
FIG. 17 represents the effect of PI3K-inhibitor, LY294002 on the cell viability treated with PEP 1. The increased cell viability after treating with PEP 1 decreased when treated with LY294002.

### DETAILED DESCRIPTION OF THE INVENTION

Since the present invention can have adaptability for diverse transformation and examples of practical application, below is a more detailed description of the present invention. Nevertheless, this is no means to limit the form of practical application; it should be understood that the intention is to include the concept and the extent of technology in all of the transformation, equivalents to alternatives. In describing the present invention, if any detailed description about the prior art is considered to deteriorate the fundamental principles of the present invention, the description will be omitted.

A telomere is known as a repetitive sequence of genetic material at the ends of chromosomes that prevent chromosomes from damage or merging of other chromosomes. The length of a telomere is shortened at each cell division, and after a certain number of cell division, the telomere length is extremely shortened to the extent in which the cell stops dividing and dies. On the other hand, the elongation of telomeres is known to extend the life span of a cell. For an example, cancer cells excrete an enzyme called telomerase, which prevents shortening of telomeres, thus resulting in proliferation of cancer cells. The present invention was accomplished upon the discovery of telomerase-derived peptides with anti-inflammatory effects.

In one embodiment of the present invention, a peptide with anti-inflammatory activities is provided. The peptide comprises at least one amino acid sequence of SEQ ID NO: 1, the peptide has above 80% homology with above-mentioned sequence, or the peptide is a fragment of the above-mentioned peptides.

Peptide with anti-inflammatory activity in the present invention is a peptide having an amino acid sequence to SEQ ID NO: 1. Peptide of SEQ ID NO: 1 is a peptide consisting of 16 amino acids in the location of telomerase- [611-626].

### SEQ ID NO: 1 EARPALLTSRLRFIPK

In one embodiment of the present invention, a polynucleotide that codes a peptide with anti-inflammatory activities is provided. The polynucleotide codes a peptide comprising at least one amino acid sequence of SEQ ID NO: 1, a peptide having above 80% homology with above-mentioned sequence, or a peptide being a fragment of the above-mentioned peptides. The polynucleotide mentioned above enables production of the peptides in large quantities. For example, cultivation of vectors that include polynucleotides encoding peptides allows production of peptides in large quantities.

The peptides disclosed herein can include a peptide comprising amino acid sequence above 80%, above 85%, above 90%, above 95%, above 96%, above 97%, above 98%, or above 99% homology. Moreover, the peptides disclosed in the present invention can include a peptide comprising SEQ ID NO: 1 or its fragments, and a peptide with more than 1 transformed amino acid, more than 2 transformed amino acid, more than 3 transformed amino acid, more than 4 transformed amino acid, more than 5 transformed amino acid, more than 6 transformed amino acid, or more than 7 transformed amino acid.

In the present specification and claims, the terms "homology" and "sequence identity" are used interchangeably to indicate the degree of sequence overlap between two amino acid (or if relevant: nucleic acid) sequences.

Unless otherwise stated the term "Sequence identity" for peptides as used herein refers to the sequence identity calculated as (*n_{ref} - n_{dif}*)·100/*n_{ref},* wherein *n_{dif}* is the total number of non-identical residues in the two sequences when aligned so that a maximum number of amino acids are identical and wherein *n_{ref}* is the number of residues in the shortest of the sequences. Hence, the DNA sequence agtcagtc will have a sequence identity of 75% with the sequence aatcaatc (*n_{dif}*=2 and *n_{ref}*=8).

In some embodiments the sequence identity is determined by conventional methods, e.g., Smith and Waterman, 1981, Adv. Appl. Math. 2:482, by the search for similarity method of Pearson & Lipman, 1988, Proc. Natl. Acad. Sci. USA 85:2444, using the CLUSTAL W algorithm of Thompson et al., 1994, Nucleic Acids Res 22:467380, by computerized implementations of these algorithms (GAP, BESTFIT, FASTA, and TFASTA in the Wisconsin Genetics Software Package, Genetics Computer Group). The BLAST algorithm (Altschul et al., 1990, Mol. Biol. 215:403-10) for which software may be obtained through the National Center for Biotechnology Information www.ncbi.nlm.nih.gov/) may also be used. When using any of the aforementioned algorithms, the default parameters for "Window" length, gap penalty, etc., are used.

In one embodiment of the present invention, changes in amino acid sequence belong to the modification of peptide's physical and chemical characteristics. For example, amino acid transformation can be performed by improving thermal stability of the peptide, altering substrate specificity, and changing the optimal pH.

In one embodiment of the present invention, a peptide comprising amino acid sequence of SEQ ID NO: 1, a peptide comprising of amino acid sequence above 80% homology with above-mentioned sequence or a peptide fragment of above-mentioned peptides is preferably made of 30 or less amino acids.

In one embodiment of the present invention, a peptide comprising amino acid sequence of SEQ ID NO: 1, a peptide comprising of amino acid sequence above 80% homology with above-mentioned sequence or a peptide fragment of above-mentioned peptides comprises a peptide originates from telomerase, more specifically, telomerase of *Homo sapiens.*

The term "amino acid" herein includes not only the 22 standard amino acids that are naturally integrated into peptide but also the D-isomers and transformed amino acids. Therefore, in a specific embodiment of the present invention, a peptide herein includes a peptide having D-amino acids. On the other hand, a peptide may include non-standard amino acids such as those that have been post-translationally modified. Examples of post-translational modification include phosphorylation, glycosylation, acylation(including acetylation, myristorylation, plamitoylation), alkylation, carboxylation, hydroxylation, glycation, biotinylation, ubiquitinylation, transformation in chemical properties (e.g. β-removing deimidation, deamidation) and structural transformation (e.g. formation of disulfide bridge). Also, changes of amino acids are included and the changes of amino acids occur due to chemical reaction during the combination process with crosslinkers for formation of a peptide conjugate.

A peptide disclosed herein may be a wild-type peptide that has been identified and isolated from natural sources. On the other hand, when compared to peptide fragments of SEQ ID NO: 1, the peptides disclosed herein may be artificial mutants that comprise one or more substituted, deleted and/or inserted amino acids. Amino acid alteration in wild-type polypeptide - not only in artificial mutants - comprises conservative substitution of amino acids that do not influence protein folding and or activation. Examples of conservative substitution belong to the group consisting of basic amino acids (arginine, lysine and histidine), acidic amino acids (glutamic acid and aspartic acid), polar amino acids (glutamine and asparagines), hydrophobic amino acids (leucine, isoleucine, valine and methionine), aromatic amino acids (phenylalanine, tryptophan and tyrosine), and small amino acids (glycine, alanine, serine, and threonine). The amino acid substitutions that do not generally alter the specific activity are known in the art of the present invention. Most common occurred alteration are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and the opposite alterations. Another example of conservative substitutions are shown in the following table 1.

**Table 1**

| Original amino acid | Examples of residue substitution | Preferable residue substitution |
|---|---|---|
| Ala (A) | val; leu; ile | Val |
| Arg (R) | lys; gln; asn | Lys |
| Asn (N) | gln; his; asp, lys; arg | Gln |
| Asp (D) | glu; asn | Glu |
| Cys (C) | ser; ala | Ser |
| Gln (Q) | asn; glu | Asn |
| Glu (E) | asp; gln | Asp |
| Gly (G) | ala | Ala |
| His (H) | asn; gln; lys; arg | Arg |
| Ile (I) | leu; val; met; ala; phe; norleucine | Leu |
| Leu (L) | norleucine; ile ; val; met; ala; phe | Ile |
| Lys (K) | arg; gln; asn | Arg |
| Met (M) | leu; phe; ile | Leu |
| Phe (F) | leu; val; ile; ala; tyr | Tyr |
| Pro (P) | ala | Ala |
| Ser (S) | thr | Thr |
| Thr (T) | ser | Ser |
| Trp (W) | tyr; phe | Tyr |
| Tyr (Y) | trp; phe ; thr; ser | Phe |
| Val (V) | ile; leu; met; phe; ala; norleucine | Leu |

The substantial transformation of the biological properties of peptides are performed by selecting significantly different substitution in the following efficacies: (a) the efficacy in maintaining the structure of the polypeptide backbone in the area of substitution, such as sheet or helical three-dimensional structures, (b) the efficacy in maintaining electrical charge or hydrophobicity of the molecule in the target area, or (c) the efficacy of maintaining the bulk of the side chain. Natural residues are divided into groups by general side chain properties as the following:
(1) hydrophobicity: Norleucine, met, ala, val, leu, ile;
(2) neutral hydrophilicity: cys, ser, thr;
(3) acidity: asp, glu;
(4) basicity: asn, gln, his, lys arg;
(5) residue that affects chain orientation: gly, pro; and
(6) aromaticity: trp, tyr, phe.

Non-conservative substitutions may be performed by exchanging a member of the above classes to a different class's. Any cysteine residues that are not related in maintaining the proper three-dimensional structure of the peptide can typically be substituted into serine, thus increasing the oxidative stability of the molecule and preventing improper crosslinkage. Conversely, improvement of stability can be achieved by adding cysteine bond(s) to the peptide.

Altered types of amino acids variants of peptides are those that antibody glycosylation pattern changed. The term "change" herein relates to deletion of carbohydrate residues and/or addition of at least one glycosylated residues that do not exist within a peptide. Glycosylation in peptides are typically N-connected or O-connected. The term "N-connected" herein relates to that carbohydrate residues are attached to the side chain of asparagine residues. As tripeptide sequences, asparagine-X-serine and asparagine-X-threonine (where the X is any amino acid except proline) are the recognition sequence for attaching carbohydrate residue enzymatically to the side chain of asparagine. Therefore, with the presence of one of these tripeptide sequences in a polypeptide, the potential glycosylation sites are created. "O-connected glycosylation" means attaching one of sugar N-acetylgalactosamine, galactose, or xylose to hydroxyl amino acids. The hydroxyl amino acids are most typically serine or threonine, but 5-hydroxyproline or 5-hydroxylysine can be used. Addition of glycosylation site to a peptide is conveniently performed by changing amino acid sequence to contain tripeptide sequence mentioned above (for N-linked glycosylation sites). These changes may be made by addition of at least one serine or theronine residues to the first antibody sequence, or by substitution with those residues (for O-linked glycosylation sites).

In one one aspect of the present diclosure, a polynucleotide is a nucleic acid molecule that can be spontaneous or artificial DNA or RNA molecules, either single-stranded or doublestranded. The nucleic acid molecule can be one or more nucleic acids of same type (for example, having a same nucleotide sequence) or nucleic acids of different types. The nucleic acid molecules comprise one or more DNA, cDNA, decoy DNA, RNA, siRNA, miRNA shRNA, stRNA, snoRNA, snRNA PNA, antisense oligomer, plasmid and other modified nucleic acids, but not limited to those.

A HMGB1 protein is known as a cytokine. It first undergoes acetylation and translocation to cytoplasm by external stimulation. Then it is secreted out of the cell, therefore serving the role of inflammation-causing cytokine. Because when one has an inflammation due to such activity, HMGB1 protein is secreted out of the cell, and patients with inflammatory diseases such as Churg strauss syndrome, rheumatoid arthritis and Sjogren's syndrome will present with elevated serum levels of HMGB1. Hence, if nucleus contains large amount of HMGB1 even when there is a stimulus that causes inflammation, it is suggestive of the fact that HMGB1 is not being secreted out of the cell, which means inflammation is being suppressed.

In one embodiment of the present invention, when treated a cell with a peptide comprising amino acid sequence of SEQ ID NO: 1, a peptide having above 80% homology of amino acid sequence with above-mentioned sequence, or a fragment of the above-mentioned peptides, amount of HMGB1 within the nucleus increases. This represents that the peptides mentioned above have excellent inflammation preventive or suppressive effects.

Also, in specific embodiments of the present invention, a peptide comprising amino acid sequence of SEQ ID NO: 1, a peptide having above 80% homology of amino acid sequence with above-mentioned sequence, or a fragment of the above-mentioned peptides, has an advantage in that it has high feasibility due to its low toxicity within a cell.

In the present invention, an "inflammatory disease" is a broad indication that refers to any disease that designates inflammation as a main cause or inflammation caused by disease. Specifically, an inflammatory disease includes (1) general or localized inflammatory disease (for example, allergies; immune-complex disease; hayfever; hypersensitive shock; endotoxin shock; cachexia, hyperthermia; granulomatosis; or sarcoidosis); (2) gastro-intestinal related diseases (for example, appendicitis; gastric ulcer; duodenal ulcer; peritonitis; pancreatitis; ulcerative, acute, or ischemic colitis; cholangitis; cholecystitis, steatorrhea, hepatitis, Crone's disease; or Whipple's Disease); (3) dermal related diseases (for example, psoriasis; burns; sunburns; dermatitis; Urticarial warts or wheal); (4) vascular related diseases (for example, angiitis; vasculitis; endocarditis; arteritis; atherosclerosis; thrombophlebitis; pericarditis; congestive heart failure; myocarditis; myocardial ischemia; periarteritis nodosa; recurrent stenosis; Buerger's disease; or rheumatic fever); (5) respiratory diseases (for example, asthma; epiglottitis; bronchitis; emphysema; rhinitis; cystic fibrosis; interstitial pneumonitis; COPD(chronic obstructive pulmonary disease); adult respiratory distress syndrome; coniosis; alveolitis; bronchiolitis; pharyngitis; pleurisy; or sinusitis); (6) bone, joint, muscle and connective tissue related diseases (for example, eosinophilic granuloma; arthritis; arthralgia; osteomyelitis; dermatomyositis; fasciitis; Paget's disease; gout; periodontal disease; rheumatoid arthritis; myasthenia gravis; ankylosing spondylitis; or synovitis); (7) urogenital disorders (for example, epididymitis; vaginitis; prostatitis; or urethritis); (8) central or peripheral nervous system related diseases (for example, meningitis; encephalitis; multiple sclerosis; cerebral infarction; cerebral embolism; Guillain-Barre syndrome; neuritis; neuralgia; spinal cord injury; paralysis; or uveitis); (9) autoimmune disease (for example, thyroiditis; lupus; Goodpasture's syndrome; allograft rejection; graft versus host disease). Treating the inflammatory component of such diseases has been a major goal of the global pharmaceutical industry for a number of decades, and a wide variety of useful treatments have been developed. Examples include the corticosteroids (a range of natural, semisynthetic and synthetic agents designed to mimic the effect of cortisol, including prednisolone, methylprednisolone, dexamethasone, betamethasone, fluticasone and so forth), cyclooxygenase inhibitors (both non-selective or cox-1 selective, such as indomethacin, sulfasalzine and aspirin, and more recently cox-2 selective, such as celecoxib), leukotriene blockers (such as monteleukast) and anti-TNFs (such as modified monoclonal neutralising antibodies, including infliximab (Remicade™) and adalimumab (Humira™), TNF receptor fusion proteins, such as etanercept (Enbrel™), as well as small molecule TNF-α synthesis inhibitors like thalidomide).

In one embodiment of the present invention, an anti-inflammatory composition for use in treating or preventing an inflammatory disease comprising a peptide as an active ingredient is provided. The peptide comprises amino acid sequence of SEQ ID NO: 1, the peptide has above 80% homology with above-mentioned sequence, or the peptide is a fragment of the above-mentioned peptides.

In one embodiment of the present invention, the anti-inflammatory composition may contain 0.1 µg/mg to 1 mg/mg, specifically 1 µg/mg to 0.5 mg/mg, more specifically 10 µg/mg to 0.1 mg/mg of a peptide comprising of amino acid sequence SEQ ID NO: 1, a peptide comprising of amino acid sequence above 80% homology with above-mentioned sequence, or peptide fragment of above-mentioned peptides. When the peptide is contained in the above mentioned range, all the safety and stability of the composition may be satisfied and appropriate in terms of cost-effectiveness.

In one embodiment of the present invention, the composition may have application with all animals including human, dog, chicken, pig, cow, sheep, guinea pig, and monkey.

In one embodiment of the present invention, the pharmaceutical composition for the use of treatment or prophylaxis of inflammatory disease with an active ingredient that is comprised of a peptide consisting of an amino acid of SEQ ID NO: 1, a peptide comprising of amino acid sequence above 80% homology with above-mentioned sequence, or peptide fragment of SEQ ID NO: 1, is provided. In one embodiment of the present invention, the pharmaceutical composition may be administered through oral, rectal, transdermal, intravenous, intramuscular, intraperitoneal, in the bone marrow, epidural or subcutaneous means.

Forms of oral administration may be, but not limited to, tablets, pills, soft or hard capsules, granules, powders, solution, or emulsion. Forms of non-oral administration may be, but not limited to, injections, drips, lotions, ointments, gels, creams, suspensions, emulsions, suppository, patch, or spray.

In one embodiment of the present invention, the pharmaceutical composition, if necessary, may contain additives, such as diluents, excipients, lubricants, binders, disintegrants, buffers, dispersants, surfactants, coloring agents, aromatics or sweeteners. In one embodiment of the present invention, the pharmaceutical composition can be manufactured by conventional methods of the industry in the art.

In one embodiment of the present invention, the active ingredient of the pharmaceutical composition may vary according to the patient's age, sex, weight, pathology and state, administration route, or prescriber's judgment. Dosage based on these factors is determined within levels of those skilled in the art, and the daily dose for example may be, but not limited to, 0.1 µg / kg / day to 1 g / kg / day, specifically 1 µg / kg / day to 10 mg / kg / day, more specifically the 10 µg / kg / day to 1 mg / kg / day, more specifically the 50 µg / kg / day to 100 µg / kg / day. In one embodiment of the present invention, the pharmaceutical composition may be administered, but not limited to, 1 to 3 times a day.

In one embodiment of the present invention, a skin external composition for improvement or prevention of skin inflammation is provided. The skin external composition may contain an active ingredient that is a peptide comprising of an amino acid sequence of SEQ ID NO: 1, a peptide comprising of amino acid sequence above 80% homology with above-mentioned sequence, or a peptide fragment of above-mentioned peptides.

In another embodiment of the present invention, a cosmetic composition for improvement or prevention of skin inflammation is provided. The cosmetic composition may contain an active ingredient that is a peptide comprising of an amino acid sequence of SEQ ID NO: 1, a peptide comprising of amino acid sequence above 80% homology with above-mentioned sequence, or peptide fragment of above-mentioned peptides.

In one embodiment of the present invention, external application composition or cosmetic composition may be provided in all forms appropriate for topical applications. For example, forms can be provided as solutions, emulsions obtained by dispersion of oil phase in water, emulsion obtained by dispersion of water in oil phase, suspension, solid, gel, powder, paste, foam or aerosol. These forms can be manufactured by conventional methods of the industry in the art.

In one embodiment of the present invention, the cosmetic composition may include, within levels that will not harm the main effect, other ingredients that can desirably increase the main effect. In one embodiment of the present invention, the cosmetic composition may additionally include moisturizer, emollient agents, surfactants, UV absorbers, preservatives, fungicides, antioxidants, pH adjusting agent, organic or inorganic pigments, aromatics, cooling agent or antiperspirant. The formulation ratio of the above-mentioned ingredients can be decided by those skilled in the art within levels that will not harm the purpose and the effects of the present invention, and the formulation ratio based on total weight of the cosmetic composition can be 0.01 to 5% by weight, specifically 0.01 to 3% by weight.

In one embodiment of the present invention, a food composition for inflammation prevention or suppression is provided. The food composition may contain with an active ingredient that is a peptide comprising of an amino acid sequence of SEQ ID NO: 1, a peptide comprising of amino acid sequence above 80% homology with above-mentioned sequence, or peptide fragment of above-mentioned peptides.

In one embodiment of the present invention, food composition is not limited to forms, but for example may be granules, powder, liquid, and solid forms. Each form can be formed with ingredients commonly used in the industry appropriately chosen by those skilled in the art, in addition to the active ingredient, and can increase the effect with other ingredients.

Decision for dosage on the above-mentioned active ingredient is within the level of those skilled in the art, and daily dosage for example may be 1 µg / kg / day to 10 mg / kg / day, more specifically the 10 µg / kg / day to 1 mg / kg / day, more specifically the 50 µg / kg / day to 100 µg / kg / day, but not limited to these numbers and can vary according to age, health status, complications and other various factors.

In one embodiment of the present invention, a use of prevention or treatment of inflammatory disease with a peptide comprising of an amino acid sequence of SEQ ID NO: 1, a peptide comprising of amino acid sequence above 80% homology with above-mentioned sequence, or peptide fragment of above-mentioned peptides, is provided.

In one embodiment of the present invention, the method of prevention or treatment of inflammatory disease with applying peptides mentioned above in patients is provided.

In one embodiment of the present invention, a kit for prophylaxis or treatment of inflammatory diseases is provided. The kit may contain: a peptide with anti-inflammatory activity or a composition comprising of the peptide, wherein the peptide comprises amino acid sequence of SEQ ID NO: 1, the peptide has above 80% homology with above-mentioned sequence, or the peptide is a fragment of the above-mentioned peptides; and instructions including at least one of administration dose, administration route, administration frequency, and indication of the peptide or composition.

The terms used herein is intended to be used to describe the embodiments, not to limit the present invention. Terms without numbers in front are not to limit the quantity but to show that there may be more than one thing of the term used. The term "including", "having", "consisting", and "comprising" shall be interpreted openly (i.e. "including but not limited to").

Mention of range of numbers is used instead of stating separate numbers within the range, so unless it is explicitly stated, each number can be read as separate numbers integrated herein. The end values of all ranges are included in the range and can be combined independently.

Unless otherwise noted or clearly contradicting in context, all methods mentioned herein can be performed in the proper order. The use of any one embodiment and all embodiment, or exemplary language (e.g., that use "like ∼"), unless included in the claims, is used to more clearly describe the present invention, not to limit the scope of the present invention. Any language herein outside of the claims should not be interpreted as a necessity of the present invention. Unless defined otherwise, technical and scientific terms used herein have meaning normally understood by a person skilled in the art that the present invention belongs to.

The preferred embodiments of the present invention are the best mode known to the inventors to perform the present invention. It may become clear to those skilled in the art after reading the statements ahead of the variations in the preferred embodiments. The present inventors hope that those skilled in the art can use the variations adequately and present invention be conducted in other ways than listed herein. Thus, the present invention, as allowed by the patent law, includes equivalents, and variations thereof, of the key points of the invention stated in the appended claims. In addition, all possible variations within any combination of the above-mentioned components are included in the present invention, unless explicitly stated otherwise or contradicting in context.

Tumor necrosis factor (TNF), particularly TNF-α, is known to be released from inflammatory cells and cause various cytotoxic reactions, immunological reactions and inflammatory reactions. TNF-α is known to be involved in the occurrence and prolongation of many inflammatory and autoimmune diseases and further cause serious septicemia and septic shock when it is released into the blood and acts systemically. Because TNF-α is a factor associated widely with the immune system of a living body, the development of agents inhibiting TNF-α is actively carried out. TNF-α is biosynthesized in an inactive form and becomes an active form by being cleaved by protease; the enzyme responsible for the activation is called a tumor necrosis factor-converting enzyme (TACE). Thus, a substance inhibiting this TACE can treat, improve, or prevent diseases, pathologic conditions, abnormal conditions, troubles, adverse symptoms and the like ascribed to TNF-α(KR2011-0060940A). High-mobility group box 1(HMGB1) protein exists in high concentrations in thymus, lymph nodes, testes, and in fetal liver, and with exception to liver and brain cells, usually exists inside of the nucleus. The said HMGB1 protein has 3 domains consisting of A-box, B-box, and C-terminal.

It was reported by Tracey et al., 1999 that HMGB1 protein has a role as a cytokine which induces inflammation, and the mechanism of said HMGB1's inflammation induction is by an external stimulus causing acetylation of HMGB1 which then moves from the nucleus into the cytoplasm. Afterward, it is known to be secreted out of the cell, or secreted out from the cell in necrosis. (Bonaldi T et al., EMBO J, (22)5551-60, 2003).

The invention is further described by the figures, the following examples and experiments, which are solely for the purpose of illustrating specific embodiments of this invention, and are not to be construed as limiting the scope of the invention in any way.

### EXAMPLE 1

### Synthesis of PEP-1 and Measurement of anti-inflammatory activities of PEP-1 (SEQ ID NO: 1)

### Experiment 1-1: Synthesis of PEP-1 (SEQ ID NO: 1)

A peptide comprised of 16 amino acids with the chemical structure 1 as below having the sequence SEQ ID: 1 (PEP-1) derived from human telomerase was synthesized:

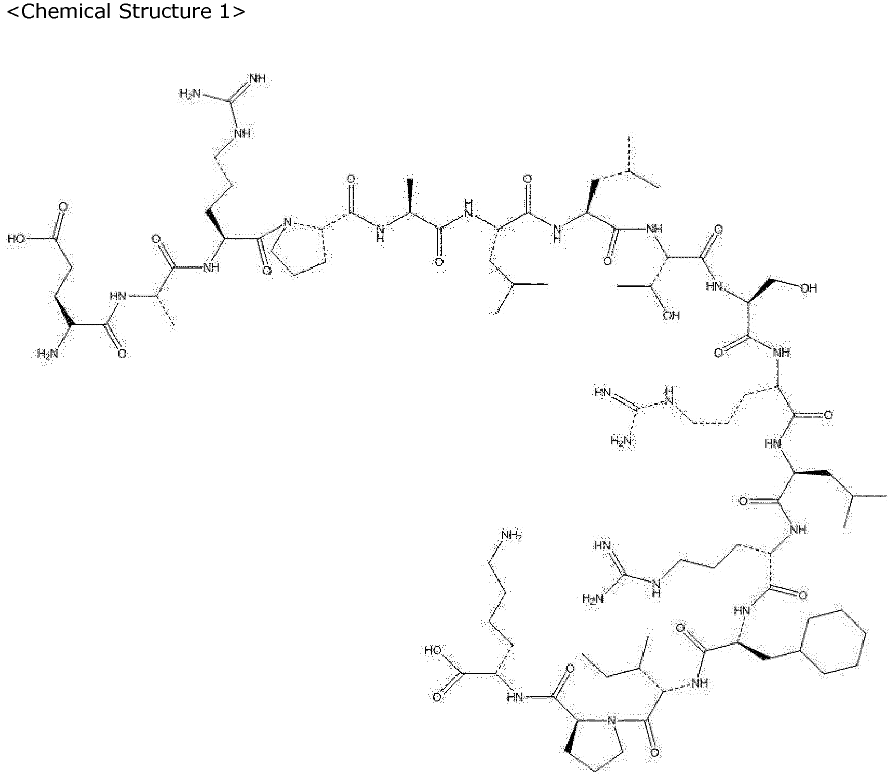

SEQ ID NO: 1 (PEP-1) was synthesized according to the existing method of solid phase peptide synthesis. In detail, the peptides were synthesized by coupling each amino acid from C-terminus through Fmoc solid phase peptide synthesis, SPPS, using ASP48S (Peptron, Inc., Daejeon ROK). Those peptides with their first amino acid at the C-terminus being attached to resin were used as follows:
NH₂-Lys(Boc)-2-chloro-Trityl Resin
NH₂-Ala-2-chloro-Trityl Resin
NH₂-Arg(Pbf)-2-chloro-Trityl Resin

All the amino acid materials to synthesize the peptide were protected by Fmoc at the N-terminus, and the amino acid residues were protected by Trt, Boc, t-Bu (t-butylester), Pbf (2,2,4,6,7-pentamethyl dihydro-benzofuran-5-sulfonyl) that can be dissolved in acid. Such as:
Fmoc-Ala-OH, Fmoc-Arg(Pbf)-OH, Fmoc-Glu(OtBu)-OH, Fmoc-Pro-OH, Fmoc-Leu-OH, Fmoc-Ile-OH, Fmoc-Phe-OH, Fmoc-Ser(tBu)-OH, Fmoc-Thr(tBu)-OH, Fmoc-Lys(Boc)-OH, Fmoc-Gln(Trt)-OH, Fmoc-Trp(Boc)-OH, Fmoc-Met-OH, Fmoc-Asn(Trt)-OH, Fmoc-Tyr(tBu)-OH, Fmoc-Ahx-OH, Trt-Mercaptoacetic acid.

HBTU[2-(1H-Benzotriazole-1-yl)-1,1,3,3-tetamethylaminium hexafluorophosphate] / HOBt [N-Hydroxybenzotriazole] /NMM [4-Methylmorpholine] were used as the coupling reagents. In 20% of DMF, piperidine was used to remove Fmoc. In order to remove the protection from residue or to separate the synthesized peptide from Resin, cleavage cocktail [TFA (trifluoroacetic acid) /TIS (triisopropylsilane) / EDT (ethanedithiol) / H₂O=92.5/2.5/2.5/2.5] was used.

Synthesized the peptide by using the solid phase scaffold combined to starting amino acid with the amino acid protection, reacting the corresponding amino acids separately, washing with solvent and deprotected, and repeating the process. After cutting off the synthesized peptide from the resin, it was purified by HPLC and verify for synthesis by MS, and then freeze-dried.

Specific synthesis process of PEP 1 is described by the following.

### 1) Coupling

Melted the amino acid (8 equivalent) protected with NH₂-Lys(Boc)-2-chloro-Trityl Resin, and coupling agent HBTU(8 equiv.)/HOBt(8equiv.)/NMM(16 equiv.) and added to DMF, then let react in room temperature for 2 hours, then washed with DMF, MeOH, and DMF in that order.

### 2) Fmoc deprotection

Added 20% piperidine in DMF and reacted in room temperature for 5 minutes 2 times, then washed with DMF, MeOH, and DMF in that order.
3) Make the basic framework of peptide by repeating reactions 1 and 2 repeatedly.
4) Cleavage: Add Cleavage Cocktail to the completely synthesized peptide and separated the peptide from the resin.
5) Add cooling diethyl ether into obtained mixture, and then centrifugation is used to precipitate gathered peptide.
6) After purification by Prep-HPLC, check the molecular weight by LC/MS and lyophilize to produce in powder form.

### EXAMPLE 2

### Anti-inflammatory activity measurement of PEP-1

### Cell lines culture

Raw 264.7macrophage cell (KCBL, 40071) from Korea Cell Bank was maintained in Dulbecco's modified Eagle's medium (DMEM; PAA, Austria) containing 10% fetal bovine serum (FBS; Gibco Laboratories), 100 unit/mL of streptomycin, and penicillin (Gibco Laboratories) at 37°C with 5% CO. Raw264.7 cells were seeded into a 96-well plate at a density of 1 x 10⁶ cells/mL and incubated overnight.

On the following day, the medium was replaced with fresh medium and 5 µg/mL of peptide (obtained as described in Experiment example 1) was added to the cells. After 30 min incubation of cells with the peptide 50 µL of LPS (to a final concentration of 1 µg/mL) was added, and cells were incubated for additional 24 hr. The experimental sample with the induction of inflammatory response was treated with 1 µg/mL mL lipopolysaccharide (LPS; Sigma, USA) and control sample was treated with phosphate buffered saline (PBS; pH 7.2). The supernatant samples from each condition was collected in eppendorf tubes and subjected to further analysis.

### Experiment 2-1. NO level analysis

The level of nitric oxide (NO) was measured in Raw 264.7 cell (1 x 10⁶ cell/ml) using Griess reagent system (Promega, USA). Culture medium of 50 µl was added to a 96-well plate and Griess reagent I (NED) solution and Griess reagent II (Sulfaniliamide solution) are added in the same amount. After 10 min incubation of cells with the reagents, the optical density at 540nm was measured within 30 min using a microplate reader (Molecular Devices, USA). The concentration of NO was calculated by using a standard curve (0∼100 µM) of sodium nitrite.

As shown in Table 2 below, stimulation of cells with LPS increased the expression of NO, but in co-treatment with LPS and PEP-1, the expression level of NO mentioned above decreased. NO is produced during inflammation, and the result showing PEP-1 reduced NO level to 65% of the control strongly support the anti-inflammatory effect of PEP-1.

**Table 2. The measurement of anti-inflammatory effect of human telomerase derived PEP 1**

| Test sample | | NO Expression Level of control (%) | Decreased NO Expression Level (%) |
|---|---|---|---|
| PBS | | 0 | - |
| LPS 1µ/mL | PBS | 100 | 0 |
| | PEP 1 (0.5µg/mL) | 35 | 65 |

### Experiment 2-2. Analysis of cytokine inhibitory effect

To investigate the effect of PEP-1 on inhibiting proinflammatory cytokine production RAW 264.7 cell were pre-treated with PEP 1 at a concentration of 5 µg/mL challenged with LPS at a concentration of 1 µg/mL, and cells were further incubated for 24 hr. The supernatant samples containing cell culture medium was collected and analyzed for the cytokine levels using ELISA kits (eBioscience, San Diego).

96 wells plates were coated with 100 µL of capture antibodies (diluted in coating buffer to the concentration recommended by manufacturer's protocol) overnight at 4°C. Then, after washing the plates 5 times, 200 µL of assay diluents was added to each well and incubated for 1 hr at room temperature for blocking. After washing each well with wash buffer five times, cell culture sample or each cytokine standard protein sample was diluted and 100 µL of each added into each well. The plate containing samples were incubated overnight at 4°C. Then, after washing the plate five times with the wash buffer, 100 µL of secondary antibody conjugated to avidin was added and incubated for 1 hr at room temperature.

Following incubation with the secondary antibody, the plate was washed five times and incubated with 100µL of avidin-HRP (BD Bioscience) for 30 min at room temperature. After washing the plate seven times, 100 µL of TMB solution (Pierce) was added and incubated for 15 min at room temperature. The reaction was stopped by adding 50 µl of 2N H₂SO₄ in each well The optical density at 450nm was measured using a microplate reader. Statistical analysis was performed by variance analysis using ANOVA procedure of SPSS program, and verified the significance between analyses using Duncan's multiple range test.

### Experiment 2-3. IL-6 secretion measurement

As shown in Table 3 below, treatment with LPS alone increased the cytokine IL-6 (interleukin-6) secretion. However, co- treatment with LPS and PEP-1 showed a decrease in the level of the proinflammatory cytokine IL-6 secretion. More importantly, after the treatment with PEP-1, the level of proinflammatory cytokine secretion decreased by more than 70%, which indicates a robust anti-inflammatory effect of PEP-1.

**Table 3. Cytokine IL-6 production inhibition by PEP-1**

| Test sample | | cytokine IL-6 production | |
|---|---|---|---|
| | | % of control | inhibition % |
| PBS | | 0 | - |
| LPS 1µg/ml | PBS | 100 | 0 |
| | PEP 1 (5µg/ml) | 28 | 72 |

### Experiment 2-4. HMGB1, TNF-α, COX-2 expression inhibition

Protein expression level was determined by Western blot analysis. Cells grown in PEP-1 containing medium were washed with PBS, treated with 0.05% trypsin-EDTA, and collected by centrifugation. The collected cells were dissolved in an appropriate volume of lysis buffer. Intracellular debris was pelleted by centrifugation, and equal amount of protein from each sample was separated by SDS-polyacrylamide gel electrophoresis. The separated protein was transferred to nitrocellulose membrane (Schleicherand Schuell, Keene, NH, USA), then was tested for the antibody specific for each protein.. The membrane was incubated with ECL (enhanced chemiluminoesence) solution(Amersham Life Science Corp., Arlington Heights, IL, USA), exposed to X-ray, and the level of protein expression was analyzed according to the exposure level shown on the X-ray film.

Western blot analysis was performed to determine the inhibitory effect of PEP-1 on the cytokine protein expression. As shown in Table 4 below, stimulation of cells with LPS increased the expression of cytokines; HMGB1, TNF-α and COX. However, if cells were treated with both LPS and PEP-1, the expression level of pro-inflammatory cytokines mentioned above decreased. The result showing the treatment with PEP-1 decreased pro-inflammatory cytokine levels by more than 70% provide strong evidence supporting the anti-inflammatory effect of PEP-1.

**Table 4. The measurement of inhibitory effect of PEP-1 on pro-inflammatory cytokine expression level.**

| Test sample | | Cytokine Expression Level (band intensity) % of control | | |
|---|---|---|---|---|
| | | HMGB1 | TNF-α | COX-2 |
| PBS | | - | - | - |
| LPS 1µg/ml | PBS | 100 | 100 | 100 |
| | PEP 1 (5µg/ml) | 30 | 25 | 22 |

### EXAMPLE 3

### Investigation of the inhibitory effect of PEP-1 on TNF-α level in HepG2 cells

### Experiment 3-1: Cell Culture

PBMC (peripheral blood mononuclear cell) was separated from the blood samples (50ml) collected from healthy subjects using Ficoll-PaqueTM PLUS (GE Healthcare Life Sciences, Piscataway, NJ, USA). PBMCs were then enriched in complete RPMI 1640 medium containing 20% of human serum, followed by transferring to 100mm polystyrene cell culture plate coated with human serum for 30 mins. After 2 hr incubation at 37 °C and 5% CO₂, the monocytes were detached from the bottom of cell culture plate using cold PBS (Phosphate Buffered Saline) (Gibco/Life Technologies, Carlsbad, CA, USA), and 1 × 10⁵ cells were cultured in each well of 96-well plate in RPMI 1640 medium (supplemented with penicillin-streptomycin; 100 mg/ml, human serum; 20%) over night.

For Luciferase Analysis, HEK293/null (human embryonic kidney) cells and HEK293/TRL stably expressing TLR2 (toll-like receptor2) obtained from Seoul National University School of Dental Medicine were used.. One day before the luciferase experiment, 2.5 × 10⁵ cells were seeded into each well of 12-well plate and cultured overnight in DMEM (Dulbecco's modified Eagle's medium) medium (supplemented with blasticidin; 10 µg/ml, fetal bovine serum; 10%)(Invitrogen/Life Technologies, Carlsbad, CA, USA)

### Experiment 3-2: Cytokine Assay

To see the effect of PEP-1 on TNF-α level in terms of protein expression level, ELISA (enzyme linked immunosorbent assay) was performed. 1 × 10⁵ PBMC-derived monocytes were cultured in 96-well plate over night. After then, LPS (lipopolysaccharide; 10 ng/ml, Sigma) was treated for 2 hours, followed by 3 times washes with PBS. OPTI-MEM medium (Invitrogen/Life Technologies, Carlsbad, CA, USA) was then treated for an hour to induce the starvation, and 4µM of FITC (Fluorescein Isothiocyanate), FITC-TAT, PEP-1-FITC, and FITC-PEP-1 were treated for 2 hours before measuring the TNF-α level. After culturing, cell soup was collected, and the amount of TNF-α was measured using ELISA kit (R&D, Minneapolis, MN, USA) as follows:
TNF measurement uses sandwich ELISA method. 100ul of TNF-α primary antibody was added into each well of pre-coated 96-well plate, and the plate was incubated at 4 °C overnight. On next day, the plate was washed 3 times with 0.5% Tween20 wash solution for 5 min each, and then 100µl of each sample and standard solution was added and left at room temperature for 2 hrs. After washing the plate like above, 100µl of HRP-conjugated secondary antibody was added into each well and left at room temperature for 2 hrs. Again, plate was washed, and avidin/biotin was added for measuring the absorbance. TNF-α level of each sample was quantified using the standard graph calculated from the absorbance of standard solution.

PBMC-derived monocytes were stimulated with endotoxin LPS (10ng/ml) for 2hrs, starved for 1hr using OPTI-MEM, and then 4uM of FITC, FITC-TAT, PEP 1-FITC and FITC-PEP 1 were treated for 2 hrs. After incubation, TNF-α level was measured with cell culture medium using ELISA. As a result, in case of FITC and FITC-TAT, TNF-α level increased due to LPS (6.2 and 6.7ng/ml, respectively), but TNF-α level significantly decreased in case of PEP-1-FITC and FITC-PEP-1 (0.17 and 0.25 ng/ml, respectively) and the difference was statistically significant (P < 0.01) (FiG. 1).

### Experiment 3-3: Luciferase Assay

To investigate the role of PEP 1 in inflammatory response, we evaluated NF-κB expression patterns through luciferase analysis. First, we incubated HEK293/null and HEK293/TLR2 (Graduate School of Dentistry, Seoul National University) in a 12-well plate for 24 hours, so that we would get 2.5 x 10⁵ cells/well. After washing three times with PBS, medium was replaced with OPTI-MEM (Invitrogen/Life Technologies, Carlsbad, CA, USA) and incubated for 4 hours, and then a mixture of 3µl lipofectamine (Invitrogen/Life Technologies), 1µg NF-κB luciferase and 10ng renilla luciferase (Promega, Madison, WI, USA) was added into each well and again incubated for 4 hours. Lipoprotein pam3cys (10ng/ml, Sigma-Aldrich, St. Louis, MO, USA) was put into all of the wells except for those of negative control, and FITC (4 µM) and FITC-PEP 1 (4 µM) were treated for 18 hours before it was washed with PBS for three times. We confirmed the activation of NF-kB through TD-20/20 luminometer (Turner designs, Sunnyvale, CA, USA) after dissolving (lysis) of cells by putting 50µl of passive lysis buffer - provided by dual-luciferase reporter assay system (Promega) - into each well. Transfection efficacy was confirmed by cotransfection of pCMV-renilla luciferase(Promega), and we analyzed results by calibrating the luciferase values.

After transfecting NF-κB luciferase to HEK293/null and HEK293/TLR2 cell lines, pam3cys, a synthetic lipoprotein, and FITC (4 µM), a negative control were treated together, and pam3cys with FITC-PEP 1 (4 µM) were again treated together to be cultured for 18 hours. The measurement of NF-κB expression patterns by luciferase strength through lysis of cells with passive lysis buffer - provided by dual-luciferase reporter assay system (Promega)-showed that there was no difference in lipoprotein or FITC-PEP-1 treated or non-treated HEK293/null. However, when lipoprotein, an agonist of TLR2, was treated to HEK293/TLR2 cell line, NF-κB expression increased (P<0.01) compared to that in untreated, confirming occurrence of inflammatory responses. Also, NF-κB expression increased when FITC-PEP 1 was treated together compared to that in untreated; and expression decreased compared to the negative control in which lipoprotein and FITC were treated together (P<0.01) (FIG.2). Ultimately, we were able to confirm that inflammatory response that can be caused by TLR 2 is reduced when PEP 1 is treated together.

### Experiment 3-4: Reanalysis of peptides that affect levels of cytokines in THP1 cell line

As a Human acute monocytic leukemia cell line, THP-1 monocyte cell line(American Type Culture Collection (Manassas, VA, USA) was used to reconfirm the effects of PEP 1. Cells were grown at a density of 0.5-7×10⁵ cells/mL in RPMI 1640 containing 10% FBS, 0.05mM 2-mercaptoethanol, 100U/ml penicillin, 100µg/mL streptomycin, and maintained at 37°C under 5% CO₂. THP-1 cells were differentiated into macrophages by treating cells with phorbol myristate acetate (PMA) at 100 ng/mL for 24 hr at 37 °C for 24 hr.

All reagents and medium were purchased from Gibco BRL. PMA, LPS and 2-mercaptoethanol were purchased from Sigma (St. Louis, MO, USA). Peptide RIA was synthesized from Peptron (Daejeon, Republic of Korea). Reverse transcription PCR kit was purchased from Promega (Madison, WI, USA). RT² SYBR® Green qPCR Mastermix_reagents and QIAzol were purchased from QIAGEN (Valencia, CA, USA).

Following differentiation into macrophages, THP-1 cells were washed two times using complete RPMI 1640 (5 min/wash). Then, cells were treated for 4 hr. with 10ng/ml LPS and/or 4µM peptide RIA in FBS free RPMI 1640.

Total RNA samples were isolated from peptide-treated THP-1 cells by using Trizol(QIAzol) reagent and , and cDNA was synthesized by reverse transcriptase PCR using reverse transcription PCR kit from Promega following manufacturer's protocol.

Then, real-Time qPCR was performed using CFX96(Bio-Rad) instrument with SYBR Green system. Primers used in the experiments are found in Table 5. The PCR cycling conditions were 95°C for 10min for activation of HotStart DNA Taq Polymerase, followed by 45 cycles of 95°C for 10sec, 55°C for 30sec, and 72 °C for 30sec. All samples were measured in triplicate and differences in gene expression were calculated using the 2-cycle threshold method. All the data were normalized against β actin (housekeeping gene) and presented as means of +/- S.E. from at least three independent experiments.

**Table 5.Primers used for qRT-PCR analysis.**

| **Gene Name** | **DNA sequence** |
|---|---|
| *tnf-alpha* | (forward) 5'-CTATCTGGGAGGGGTCTTCC-3' |
| | (reverse) 5'-ATGTTCGTCCTGCTCACAGG-3' |
| *il-1 beta* | (forward) 5'-GGACAAGCTGAGGAAGATGC-3' |
| | (reverse) 5'-TCGTTATCCCATGAGTCGAA-3' |
| *il-6* | (forward) 5'-AAAAGTCCTGATCCAGTTCCTG-3' |
| | (reverse) 5'-TGAGTTGTCATGTCCTGCAG-3' |
| *il-8* | (forward) 5'-GTGCAGTTTTGCCAAGGAGT-3' |
| | (reverse) 5'-AATTTCTGTGTTGGCGCAGT-3' |
| *inos* | (forward) 5'-CACCATCCTGGTGGAACTCT-3' |
| | (reverse) 5'-TCCAGGATACCTTGGACCAG-3' |
| *beta actin* | (forward) 5'-AGAAAATCTGGCACCACACC-3' |
| | (reverse) 5'-GGGGTGTTGAAGGTCTCAAA-3' |

As shown in FIG. 3, the cytokines involved in the inflammatory responses decreased noticeably by treating with PEP 1.

### EXAMPLE 4

### Analysis of inflammatory response induced by amyloid-β protein

HMGB1 first undergoes acetylation and translocation to cytoplasm by external stimulation. Then it is secreted out of the cell, therefore serving the role of inflammation-causing cytokine. Because when one has an inflammation due to such activity, HMGB1 protein is secreted from the cell, and patients with inflammatory diseases such as Churg strauss syndrome, rheumatoid arthritis and Sjogren's syndrome will present with elevated serum levels of HMGB1. Hence, if nucleus contains large amount of HMGB1 even when there is a stimulus that causes inflammation, it is suggestive of the fact that HMGB1 is not being secreted out of the cell, which means inflammation is being suppressed.

### Experiment 4-1: Analysis of survival and proliferation of neural stem cells by anti-inflammatory effects of PEP-1

First of all, PEP-1 was prepared according to the manufacturing methods described in Example 1.

### Experiment 4-2: Neural stem cell culture and Amyloid-β toxicity assessment

After removing the cortex from the head of an embryonic rat that had been pregnant for 13 days, it was cultured for a week with Basic Fibroblast Growth Factor (bFGF) to obtain the neural stem cells. To analyze the effects of the amyloid-β protein on the neural stem cells, the pre-oligomerized amyloid-β protein of concentrations 0 to 40µM was treated on neural stem cells for 48 hours, then CCK-8 assay, BrdU, and TUNEL assay were used for cytotoxicity assessment (refer from BA Yankner *et al,* 1990 and KN Dahlgren *et al,* 2002). We used the same concentration of amyloid-β protein in subsequent experiments after we confirmed that cell survival was reduced to 60% when processed with 20µM of amyloid-β protein (Refer to FIG. 4 and 5).

### Experiment 4-3: Cell toxicity assessment by treatment with PEP-1

To evaluate the impact of PEP-1 on the cultured neural stem cells, the neural stem cells were firstly cultured by a well-known method (BA Yankner *et, al,* 1990 and KN Dahlgren *et al,* 2002). Then, different concentrations (0, 1, 10, 50, 100, 200µM) of PEP-1 were treated for 48 hours, followed by cell viability and proliferation assessments using MTT assay, BrdU and TUNEL assay. PEP-1's concentrations from 0 to 200µM appeared stable in the neuronal system since they did not inhibit both survival and proliferation of neural stem cells (Refer to Fig. 6 and 7).

### Experiment 4-4: Cell toxicity assessment by co-treatment of amyloid-β protein and telomerase peptide

To determine whether PEP 1 has the effect of suppressing the neurotoxicity caused by amyloid-β protein, 20µM amyloid-β protein and various concentrations of PEP-1 were co-treated for 48 hours. The cell viability and apoptosis were measured using MMT assay, CCK-8 assay, LDH assay and TUNEL assay, and neural stem cell proliferation by BrdU assay.

The results of MMT assay and CCK-8 assay confirmed that 10µM of PEP-1 began to protect neural stem cells from neurotoxicity by amyloid-β, and the most effective protection was provided in 100µM. (Refer to FIG. 8). LDH assay was carried out for assessment of the degree of cell death as another method, and we confirmed that the increase in cell death by amyloid-β decreased by PEP-1, and efficacy was seen starting at 1µM concentration (Refer to FIG. 9).

We also confirmed with BrdU assay that the decreased cell proliferation due to amyloid-β protein was restored when processed with PEP-1 (Refer to FIG. 10).

Cell mobility is a vital matter due to the nature of neural stem cell. According to the experimental results of cell mobility, we confirmed that the decreased cell proliferation due to amyloid-β protein was restored when processed with PEP-1, and that it increased even more when in 10µM concentration, compared to control. This suggests that in the future clinical trials, processing prior to stem cell transplantation may draw more effective results. (Refer to FIG. 11).

To confirm the degree of neuronal stem cells damage, TUNEL assay was performed. Neuronal stem cell death was observed to be significantly increased in 20µM amyloid-β protein treatment group, and neuronal stem cell death decreased when treated with1 to 100µM of PEP 1. (Refer to FIG. 12)

The mechanism of action of PEP-1's protective effect on apoptosis by amyloid-β protein was investigated. First, it was investigated whether PEP-1 is capable of minimizing the oxidative damage caused by amyloid-β protein. Change in generation of reactive oxygen species after treatment with amyloid-β protein and PEP-1 was observed by using DCF-DA staining (Molecular Probes, Eugene, OR). In the group in which reactive oxygen species increased due to 20µM of amyloid-β protein, the increased reactive oxygen species decreased by PEP-1 treatment (1µM, 10µM, 50µM) (Refer to FIG. 13).

### Experiment 4-5: Comparative analysis of protein expression levels between the groups treated with and without PEP-1

Protein expression level of PEP-1 treated group and untreated group was analyzed by 2D-electrophoresis technique and antibody microarray technique. Prepared 200 µg by extracting proteome from the neural stem cells cultured in Experiment 3-1 of Example 3. In addition, the group in which PEP-1 was not treated was used as the comparison group in the same condition.

2D-electrophoresis was performed using 12% acrylamide gels. First gel electrophoresis was performed at PI 4∼10N, using a gel size of 8.5x7cm. After electrophoresis, it was dyed with Colloidal Coomassie Blue, and then compared expression by using PDQuest software to analyze each spot.

Difference in the expression levels of more than 1.5 times was identified using MALDI-TOF MS (Matrix Desoprtion/lionization Time of Flight Mass Spectromestry). Among these, proteins correlated with inflammation-related signaling, such as i-NOS and HMGB-1 were identified (Refer to Table 6). The changes in protein expression levels either increased or decreased by 1.5 times by amyloid-β protein, but it was confirmed that expression level was regulated close to that of negative control when PEP-1 was added (Refer to FIG. 14).

Antibody microarray was carried out by using cell signaling kit (CSAA1, PanoramaTM Ab Microarray Cell Signaling kit), array slides were scanned by GenePix Personal 4100A scanner (Molecular Devices) and the data were analyzed by GenePix Pro 5.0 (Molecular Devices).

The Table 6 below is an analysis of expression levels of proteins associated with inflammation by 2D electrophoresis technique. The control group represents protein expression level of cells that were not treated with neither amyloid-β protein nor PEP-1. It shows increased or decreased multiple of protein expression based on the control group's expression level.

We confirmed with the results of analysis that like the suggested in Table 6 below, inflammation related protein over-expression or under-expression was controlled by PEP-1; the protein expression level was close to that of negative control group.

**Table 6**

| Protein | Negative Control | 20ug *β* -amyloid treated group (fold) | 20ug *β* - amyloid+PEP 1 treated group (fold) |
|---|---|---|---|
| HSP 70 | 1.0 | -2.3 | 1.2 |
| HSP 90 | 1.0 | -1.8 | 1.0 |
| HHGB1 | 1.0 | -1.5 | 2.8 |
| GADD 153 | 1.0 | 1.6 | 1.2 |
| i-NOS | 1.0 | 1.9 | -1.1 |
| e-NOS | 1.0 | 1.9 | -1.1 |
| Pyk2 | 1.0 | 2.0 | 1.2 |
| MAP Kinase | 1.0 | 2.2 | 1.0 |

Phosphatidylinositol 3-kinase (PI3K)/AKT signaling pathway serves a crucial role in the growth and survival of neuronal stem cells. PI3K pathway is activated by growth factors and regulatory factors, and is involved in the normal regulation of neuronal stem cell growth and survival. AKT signaling pathways disable several pro-apoptotic factors, including a well-known apoptotic signaling molecule, GSK3β.

To further investigate the anti-inflammatory effects of PEP-1, we performed Western blot on HMGB1, since it showed a major change in protein analysis. As a result, the processing of the PEP-1 increased the protein expression levels in anti-apoptotic proteins such as Ki67, pAKT, PI3K, HSTF-1 and Bcl-2, and decreased the protein expression levels of apoptotic signals such as Bax, GSK3β, Cytochrom-c, caspase-3 (Refer to FIG. 15).

HMGB1, a non-histone structure protein that binds to DNA, serves diverse roles within a cell; such as stabilizing nucleosome structure and regulating gene expression. As one of the inflammation-causing substance that is excreted in the late phase of inflammatory response, it is excreted by macrophages and monocytes when inflammation is stimulated, but when neuron is significantly damaged and leads to cell necrosis, it will be excreted out of the cell, causing an intense inflammatory response. The increase of HMGB1 by PEP-1 treatment after the decrease by amyloid-β treatment in the cytoplasm of the nerve cells reflects the fact that PEP-1 inhibits secretion of HMGB1 out of the cell caused by neuron cell death; therefore suggesting that PEP-1 has powerful anti-inflammatory effects (Refer to FIG. 15).

In addition, we investigated the response of PEP-1 to the amyloid-β aggregation. Aggregation of protein was inhibited when treated with PEP-1 (Refer to Figure16 (A)) in induction of aggregation of amyloid-β, and protein underwent degradation when PEP-1 was treated on the amyloid-β protein that was already induced for aggregation (Refer to FIG. 16(B)).

In the mechanism of action of PEP-1, we have previously confirmed the increase in cell survival signaling and decrease in apoptosis signaling of PI3K. To investigate whether these effects are direct or indirect, we treated PI3K-inhibitor, LY294002 (Promega). As a result, the increased cell viability after treating with PEP 1 decreased when treated with LY294002. Thus, we can conclude that PI3K is directly associated with PEP 1's neuroprotective effect (Refer to FIG. 17).

PEP-1 inhibits apoptosis of neural stem cells by amyloid-β protein. Also, the improvement of cell mobility of neural stem cells was confirmed, therefore suggesting a variety of possibilities in clinical application. The inhibition effects from neurotoxicity caused by beta-amyloid protein was verified by the anti-inflammatory effect of the mechanism of action of PEP 1, increased survival factors of neuro stem cells and decreased apoptotic factors, especially activation of PI3K signaling pathway and antioxidant effects.

### EXAMPLE 5

### qPCR array

### MATERIALS AND METHODS

### THP-1 cell culture

THP-1 cells (human monocytic leukemia derived cell line) were purchased from ATCC (American Type Culture Collection, Manassas, VA, USA) and cultured in RPMI-1640 (Life technologies, Carlsbad, CA, USA) medium supplemented with 10% FBS (Life technologies), 1% penicillin/streptomycin (Life technologies), and 0.05 mM 2-mercaptoethanol (Sigma-Aldrich, St. Louis, MO, USA) at 37°C in 5% CO₂. THP-1 cells that normally grow in suspension were differentiated into an adherent macrophage-like phenotype in differentiation medium (complete growth medium containing 100 ng/mL phorbol 12-myristate 13-acetate (PMA, Sigma-Aldrich)) for 24hr. For differentiation, THP-1 cells (3 x 10⁶ cells/plate, ∼95% confluency) were seeded into 10-cm tissue culture plates and incubated in differentiation medium.

### Treatment of THP-1 cells with anti-inflammatory peptide, PEP-1

Following differentiation, the macrophage-like THP-1 cells were washed twice using complete growth medium. Then, cells were treated with 10 ng/mL lipopolysaccharide (Sigma-Aldrich) and/or 4 µM PEP-1 for 4h at 37°C.

### RNA isolation and cDNA synthesis from THP-1 cells

Total RNA was extracted and purified using RNeasy mini kit from Qiagen (Valencia, CA, USA) following manufacturer's protocol. cDNAs were synthesized by reverse transcription using Reverse Transcription System from Promega (Madison, WI, USA) according to the manufacturer's protocol.

### PCR arrays

Then, cDNA samples from THP-1 cells were used as template for real-time quantitative PCR (qPCR) analysis. For qPCR analysis, RT² Profiler PCR Array kits were purchased from SABiosciences/Qiagen (Valencia, CA, USA). Four different PCR array kits analyzing separate signaling pathways used in the experiment are as follows: human signal transduction pathway finder, human inflammatory cytokines & receptors, human transcription factors, human NF-κB signaling pathway. PCR was performed with SYBR Green detection system (Qiagen) using a Bio-Rad (Mercules, CA, USA) CFX 96 real-time PCR instrument. Thermocycling conditions were: 95°C for 10 sec; 55°C for 30 sec; 95°C for 10 min; 95°C for 10sec, 55°C for 30 sec, and 72°C for 30 sec for 50 amplification cycles. Data represents the average value from three independent experiments, and % decrease was determined by the target gene expression in LPS treated samples vs. LPS + PEP-1 treated samples. Among 336 genes analyzed, only those showing statistically significant (p < 0.05, student's t-test) % decrease were shown in table 7.

### RESULTS

**Table 7. PEP-1 inhibits the genes shown in the table above (*, NF-κB target genes containing consensus NF-κB binding sites in the promoter region).**

| Signalling pathway | Gene | % Decrease by PEP 1 (LPS+PEP-1/LPS), p<0.05 |
|---|---|---|
| | TNFα * | ↓↓ 34% |
| | IL10 * | ↓↓ 32% |
| | IL1Ra * | ↓ 20% |
| | IL17C | ↓↓ 35% |
| | G-CSF * | ↓↓↓ 48% |
| | GM-CSF * | ↓↓ 29% |
| | CCL4/MIP1β * | ↓ 22% |
| | CCL26/MIP4α | ↓↓↓ 42% |
| TNF Receptor Family | TNFR11B | ↓↓↓ 38% |
| | CD40Ligand | ↓ 18% |
| Lipid biosynthesis | ACSL5 (acyl-coA synthase) | ↓ 17% |
| Apoptosis | BCL10 | ↓↓ 32% |
| NFkB | IkBα | ↓↓ 27% |

PEP-1 inhibited transcription of genes shown in Table 7 with THE % inhibition calculated as the ratio in the level of transcription between LPS treated vs. LPS+PEP-1-treated samples (THP-1 cells). Among the 336 genes analyzed, only the 13 genes in Table 7 showed statistically significant decrease following PEP-1 treatment. Those genes can be grouped into different functional categories, which include chemokines & cytokines, TNFα receptor signaling, lipid metabolism, apoptosis, and NF-κB signaling. More importantly, genes in the chemokines & cytokines category have been known as NF-κB target genes, having NF-κB consensus DNA-binding sites in their promoter regions. Taken together, data from PCR arrays support that PEP-1 may exert anti-inflammatory effects by modulating the master regulator of inflammation NF-κB, and by doing so PEP-1 can be used as anti-inflammatory therapeutic agents in a wide range of inflammatory diseases.

## Claims

1. A peptide with anti-inflammatory activity, wherein the peptide comprises
(a) the amino acid sequence of SEQ ID NO: 1, or
(b) an amino acid sequence having at least 80% sequence identity with SEQ ID NO: 1, or
(c) a fragment of the amino acid sequence of (a) or (b)
for use in treatment or prevention of inflammatory disease.

2. The peptide for the use according to claim 1, which consists of at most 30 amino acids.

3. The peptide for the use according to claim 1, which consists of amino acid sequence of SEQ ID NO: 1.

4. The peptide for the use according to claim 1, which originates from human telomerase.

5. An anti-inflammatory composition comprising a peptide defined in any one of claims 1-4 as active ingredient, for use in treatment or prophylaxis of inflammatory disease.

6. The anti-inflammatory composition for the use according to claim 5, which is a cosmetic composition for improving or preventing skin inflammation.

7. The anti-inflammatory composition for the use according to claim 5, which is a pharmaceutical composition for treatment or prophylaxis of inflammatory disease.

8. The anti-inflammatory composition for the use according to claim 5, which is a food composition for treatment or prophylaxis of inflammation.

9. The anti-inflammatory composition for the use according to claim 5 or claim 7, wherein the inflammatory disease is selected from the group consisting of (1) general or granulomatosis; or sarcoidosis); (2) gastro-intestinal related diseases (for example, appendicitis; gastric ulcer; duodenal ulcer; peritonitis; pancreatitis; ulcerative, acute, or ischemic colitis; cholangitis; cholecystitis, steatorrhea, hepatitis, Crohn's disease; or Whipple's Disease); (3) dermal related diseases (for example, psoriasis; burns; sunburns; dermatitis; Urticarial warts or wheal); (4) vascular related diseases (for example, angiitis; vasculitis; endocarditis; arteritis; atherosclerosis; thrombophlebitis; pericarditis; congestive heart failure; myocarditis; myocardial ischemia; periarteritis nodosa; recurrent stenosis; Buerger's disease; or rheumatic fever); (5) respiratory diseases (for example, asthma; epiglottitis; bronchitis; emphysema; rhinitis; cystic fibrosis; interstitial pneumonitis; COPD(chronic obstructive pulmonary disease); adult respiratory distress syndrome; coniosis; alveolitis; bronchiolitis; pharyngitis; pleurisy; or sinusitis); (6) bone, joint, muscle and connective tissue related diseases (for example, eosinophilic granuloma; arthritis; arthralgia; osteomyelitis; dermatomyositis; fasciitis; Paget's disease; gout; periodontal disease; rheumatoid arthritis; myasthenia gravis; ankylosing spondylitis; or synovitis); (7) urogenital disorders (for example, epididymitis; vaginitis; prostatitis; or urethritis); (8) central or peripheral nervous system related diseases (for example, meningitis; encephalitis; multiple sclerosis; cerebral infarction; cerebral embolism; Guillain-Barre syndrome; neuritis; neuralgia; spinal cord injury; paralysis; or uveitis); (9) autoimmune disease (for example, thyroiditis; lupus; Goodpasture's syndrome; allograft rejection; graft versus host disease).

10. A kit for use in prophylaxis or treatment of inflammatory disease comprising: a peptide according to any one of claims 1-4; and instructions including at least one of administration dose, administration route, administration frequency, and indication of the peptide or composition.

## Patentansprüche

1. Peptid mit entzündungshemmender Aktivität, wobei das Peptid umfasst:
(a) die Aminosäuresequenz von SEQ ID NO: 1, oder
(b) eine Aminosäuresequenz, die wenigstens 80 % Sequenzidentität mit SEQ ID NO: 1 aufweist, oder
(c) ein Fragment der Aminosäuresequenz von (a) oder (b)
zur Verwendung bei der Behandlung oder Vorbeugung einer entzündlichen Erkrankung.

2. Peptid zur Verwendung nach Anspruch 1, das aus höchstens 30 Aminosäuren besteht.

3. Peptid zur Verwendung nach Anspruch 1, das aus der Aminosäuresequenz von SEQ ID NO: 1 besteht.

4. Peptid zur Verwendung nach Anspruch 1, das aus humaner Telomerase stammt.

5. Entzündungshemmende Zusammensetzung, umfassend ein in einem der Ansprüche 1 bis 4 definiertes Peptid als Wirkstoff zur Verwendung bei der Behandlung oder Vorbeugung einer entzündlichen Erkrankung.

6. Entzündungshemmende Zusammensetzung zur Verwendung nach Anspruch 5, bei der es sich um eine kosmetische Zusammensetzung zur Verbesserung oder Vorbeugung einer Hautentzündung handelt.

7. Entzündungshemmende Zusammensetzung zur Verwendung nach Anspruch 5, bei der es sich um eine pharmazeutische Zusammensetzung zur Behandlung oder Vorbeugung einer entzündlichen Erkrankung handelt.

8. Entzündungshemmende Zusammensetzung zur Verwendung nach Anspruch 5, bei der es sich um eine Lebensmittelzusammensetzung zur Behandlung oder Vorbeugung einer Entzündung handelt.

9. Entzündungshemmende Zusammensetzung zur Verwendung nach Anspruch 5 oder 7, wobei die entzündliche Erkrankung aus der Gruppe ausgewählt ist, bestehend aus: (1) allgemeinen oder lokalisierten entzündlichen Erkrankungen (zum Beispiel Allergien; Immunkomplexerkrankung; Heuschnupfen; hypersensitiver Schock; Endotoxinschock; Kachexie, Hyperthermie; Granulomatose; oder Sarkoidose); (2) den Gastrointestinaltrakt betreffende Erkrankungen (zum Beispiel Appendizitis; Magengeschwür; Zwölffingerdarmgeschwür; Peritonitis; Pankreatitis; ulzerative, akute oder ischämische Colitis; Cholangitis; Cholecystitis, Steatorrhoe, Hepatitis, Morbus Crohn; oder Morbus Whipple); (3) die Haut betreffende Erkrankungen (zum Beispiel Psoriasis; Verbrennungen; Sonnenbrand; Dermatitis; Urtikaria-Warzen oder -Quaddeln); (4) die Gefäße betreffende Erkrankungen (zum Beispiel Angiitis; Vaskulitis; Endokarditis; Arteritis; Atherosklerose; Thrombophlebitis; Perikarditis; kongestive Herzinsuffizienz; Myokarditis; Myokardischämie; Periarteritis nodosa; rezidivierende Stenose; Morbus Buerger; oder rheumatisches Fieber); (5) Atemwegserkrankungen (zum Beispiel Asthma; Epiglottitis; Bronchitis; Emphysem; Rhinitis; zystische Fibrose; interstitielle Pneumonie; COPD (chronisch-obstruktive Lungenerkrankung); akutes Atemnotsyndrom bei Erwachsenen; Koniose; Alveolitis; Bronchiolitis; Pharyngitis; Pleuritis; oder Sinusitis); (6) die Knochen, die Gelenke, die Muskeln und das Bindegewebe betreffende Erkrankungen (zum Beispiel eosinophiles Granulom; Arthritis; Arthralgie; Osteomyelitis; Dermatomyositis; Fasciitis; Morbus Paget; Gicht; Parodontalerkrankungen; rheumatoide Arthritis; Myasthenia gravis; Spondylitis ankylosans; oder Synovitis); (7) Urogenitalerkrankungen (zum Beispiel Epididymitis; Vaginitis; Prostatitis; oder Urethritis); (8) Erkrankungen betreffend das zentrale oder periphere Nervensystem (zum Beispiel Meningitis; Enzephalitis; multiple Sklerose; Hirninfarkt; Hirnembolie; Guillain-Barre-Syndrom; Neuritis; Neuralgie; Rückenmarksverletzung; Paralyse; oder Uveitis); (9) Autoimmunerkrankungen (zum Beispiel Thyroiditis; Lupus; Goodpasture-Syndrom; Allotransplantat-Abstoßung; Transplantat-gegen-Wirt-Erkrankung).

10. Kit zur Verwendung bei der Vorbeugung oder Behandlung einer entzündlichen Erkrankung, umfassend: ein Peptid nach einem der Ansprüche 1 bis 4; und Anleitungen, die wenigstens eines aus der Verabreichungsdosis, dem Verabreichungsweg, der Verabreichungshäufigkeit und der Indikation des Peptids oder der Zusammensetzung beinhalten.

## Revendications

1. Peptide ayant une activité anti-inflammatoire, dans lequel le peptide comprend
(a) la séquence d'acides aminés de SEQ ID NO : 1, ou
(b) une séquence d'acides aminés ayant une identité de séquence d'au moins 80 % avec SEQ ID NO : 1, ou
(c) un fragment de la séquence d'acides aminés (a) ou (b)
pour son utilisation dans le traitement ou la prévention d'une maladie inflammatoire.

2. Peptide pour son utilisation selon la revendication 1, qui est constitué d'au plus 30 acides aminés.

3. Peptide pour son utilisation selon la revendication 1, qui est constitué de la séquence d'acides aminés de SEQ ID NO : 1.

4. Peptide pour son utilisation selon la revendication 1, qui provient de la télomérase humaine.

5. Composition anti-inflammatoire comprenant un peptide tel que défini dans l'une quelconque des revendications 1 à 4 en tant que principe actif, pour son utilisation dans le traitement ou la prophylaxie d'une maladie inflammatoire.

6. Composition anti-inflammatoire pour son utilisation selon la revendication 5, qui est une composition cosmétique pour l'amélioration ou la prévention d'une inflammation cutanée.

7. Composition anti-inflammatoire pour son utilisation selon la revendication 5, qui est une composition pharmaceutique pour le traitement ou la prophylaxie d'une maladie inflammatoire.

8. Composition anti-inflammatoire pour son utilisation selon la revendication 5, qui est une composition alimentaire pour le traitement ou la prophylaxie d'une inflammation.

9. Composition anti-inflammatoire pour son utilisation selon la revendication 5 ou la revendication 7, dans laquelle la maladie inflammatoire est sélectionnée dans le groupe constitué (1) d'une maladie inflammatoire générale ou localisée (par exemple, des allergies ; une maladie à complexes immuns ; le rhume des foins ; un choc hypersensible ; un choc endotoxinique ; la cachexie, l'hyperthermie ; la granulomatose ; ou la sarcoïdose) ; (2) de maladies associées au tractus gastro-intestinal (par exemple, l'appendicite ; un ulcère gastrique ; un ulcère duodénal ; une péritonite ; une pancréatite ; une rectocolite hémorragique, aiguë ou ischémique ; une cholangite ; une cholécystite ; une stéatorrhée, une hépatite, la maladie de Crohn ; ou la maladie de Whipple) ; (3) de maladies associées au derme (par exemple, le psoriasis ; des brûlures ; des coups de soleil ; une dermite ; des verrues ou papules oedémateuses urticariennes) ; (4) de maladies associées au système vasculaire (par exemple, une angéite ; une vasculite ; une endocardite ; une artérite ; l'athérosclérose ; une thrombophlébite ; une péricardite ; une insuffisance cardiaque congestive ; une myocardite ; une ischémie du myocarde ; une périarthrite noueuse ; une sténose récurrente ; la maladie de Buerger ; ou un rhumatisme articulaire aigu) ; (5) de maladies respiratoires (par exemple, l'asthme ; une épiglottite ; une bronchite ; un emphysème ; une rhinite ; la mucoviscidose ; une pneumonite interstitielle ; une BPCO (bronchopneumopathie chronique obstructive) ; le syndrome de détresse respiratoire adulte ; une coniose ; une alvéolite ; une bronchiolite ; une pharyngite ; une pleurésie ; ou une sinusite) ; (6) de maladies liées aux tissus osseux, articulaire, musculaire et conjonctif (par exemple, un granulome éosinophile ; l'arthrite ; une arthralgie ; une ostéomyélite ; une dermatomyosite ; une fasciite ; la maladie de Paget ; la goutte ; une parodontopathie ; la polyarthrite rhumatoïde ; la myasthénie gravis ; la spondylarthrite ankylosante ; ou une synovite) ; (7) de troubles urogénitaux (par exemple, une épididymite ; une vaginite ; une prostatite ; ou une urétrite) ; (8) de maladies liées au système nerveux central ou périphérique (par exemple, une méningite ; une encéphalite ; la sclérose en plaques ; un infarctus cérébral ; un embolisme cérébral ; le syndrome de Guillain-Barré ; une névrite ; une névralgie ; une lésion de la moelle épinière ; une paralysie ; ou une uvéite) ; (9) d'une maladie auto-immune (par exemple, une thyroïdite ; le lupus ; le syndrome de Goodpasture ; un rejet d'allogreffe ; la maladie du greffon contre l'hôte).

10. Kit pour son utilisation dans la prophylaxie ou le traitement d'une maladie inflammatoire comprenant : un peptide selon l'une quelconque des revendications 1 à 4 ; et des instructions comprenant au moins l'une de la dose à administrer, de la voie d'administration, de la fréquence d'administration, et d'une indication du peptide ou de la composition.


### SEQUENCE LISTING

<110> KAEL-GemVax Co., Ltd.
   GemVax AS
   KIM, Sang Jae
<120> ANTI-INFLAMMATORY PEPTIDES AND COMPOSITION COMPRISING THE SAME
<130> 19669PCT00
<150> KR 10-2012-0050529
   <151> 2012-05-11
<150> KR 10-2012-0050533
   <151> 2012-05-11
<150> KR 10-2012-0071989
   <151> 2012-07-02
<150> KR 10-2012-0104144
   <151> 2012-09-19
<150> KR 10-2012-0104207
   <151> 2012-09-19
<150> PCT/EP2013/055329
   <151> 2013-03-15
<160> 13
<170> PatentIn version 3.5
<210> 1
   <211> 16
   <212> PRT
   <213> Homo sapiens
<400> 1
   Glu Ala Arg Pro Ala Leu Leu Thr Ser Arg Leu Arg Phe Ile Pro Lys
   1               5                   10                  15
<210> 2
   <211> 20
   <212> DNA
   <213> Artificial Sequence
<220>
   <223> forward primer for TNF-alpha
<400> 2
   ctatctggga ggggtcttcc 20
<210> 3
   <211> 20
   <212> DNA
   <213> Artificial Sequence
<220>
   <223> reverse primer for TNF-alpha
<400> 3
   atgttcgtcc tgctcacagg 20
<210> 4
   <211> 20
   <212> DNA
   <213> Artificial Sequence
<220>
   <223> forward primer for il-1 beta
<400> 4
   ggacaagctg aggaagatgc 20
<210> 5
   <211> 20
   <212> DNA
   <213> Artificial Sequence
<220>
   <223> reverse primer for il-1 beta
<400> 5
   tcgttatccc atgagtcgaa 20
<210> 6
   <211> 22
   <212> DNA
   <213> Artificial Sequence
<220>
   <223> forward primer for il-6
<400> 6
   aaaagtcctg atccagttcc tg 22
<210> 7
   <211> 20
   <212> DNA
   <213> Artificial Sequence
<220>
   <223> reverse primer for il-6
<400> 7
   tgagttgtca tgtcctgcag 20
<210> 8
   <211> 20
   <212> DNA
   <213> Artificial Sequence
<220>
   <223> forward primer for il-8
<400> 8
   gtgcagtttt gccaaggagt 20
<210> 9
   <211> 20
   <212> DNA
   <213> Artificial Sequence
<220>
   <223> reverse primer for il-8
<400> 9
   aatttctgtg ttggcgcagt 20
<210> 10
   <211> 20
   <212> DNA
   <213> Artificial Sequence
<220>
   <223> forward primer for inos
<400> 10
   caccatcctg gtggaactct 20
<210> 11
   <211> 20
   <212> DNA
   <213> Artificial Sequence
<220>
   <223> reverse primer for inos
<400> 11
   tccaggatac cttggaccag 20
<210> 12
   <211> 20
   <212> DNA
   <213> Artificial Sequence
<220>
   <223> forward primer for beta actin
<400> 12
   agaaaatctg gcaccacacc 20
<210> 13
   <211> 20
   <212> DNA
   <213> Artificial Sequence
<220>
   <223> reverse primer for beta actin
<400> 13
   ggggtgttga aggtctcaaa 20